# EUROPEAN PATENT SPECIFICATION

(11) **EP 4 034 253 B1**
(45) Date of publication and mention of the grant of the patent: **21.01.2026**
(21) Application number: 20869832.4
(22) Date of filing: 23.09.2020
(51) Int. Cl.: A61K 31/733, A61K 31/4045, A61K 31/732, A61K 31/737, A61K 31/738, A61K 31/353, A61K 35/74, A61K 39/39, A61P 35/00, A61K 9/00, C12N 15/70, A23L 33/21, A61K 9/06, A61K 47/10, A61K 47/36, A61K 47/42

(54) **COMPOSITIONS FOR INCREASING THE EFFICACY OF IMMUNOTHERAPIES AND VACCINES**
ZUSAMMENSETZUNGEN ZUR ERHÖHUNG DER WIRKSAMKEIT VON IMMUNTHERAPIEN UND IMPFSTOFFEN
COMPOSITIONS POUR ACCROÎTRE L'EFFICACITÉ D'IMMUNOTHÉRAPIES ET DE VACCINS

(30) Priority: 23.09.2019 US 201962904395 P
(43) Date of publication of application: 03.08.2022
(60) Divisional application: 25224311.8
(73) Proprietor: The Regents Of The University Of Michigan, Ann Arbor, MI 48109-2590 (US)
(72) Inventor: MOON, James J., Ann Arbor, MI 48109-2590 (US); HAN, Kai, Ann Arbor, MI 48109-2590 (US); XU, Jin, Ann Arbor, MI 48109-2590 (US); HUANG, Xuehui, Ann Arbor, MI 48109-2590 (US)
(74) Representative: Algemeen Octrooi- en Merkenbureau B.V.
(86) International application number: PCT/US2020/052241
(87) International publication number: WO 2021/061789

(56) References cited:
- EP-A1- 3 858 368
- WO-A1-2016/053308
- WO-A1-2018/222969
- WO-A2-2016/172657
- WO-A2-2018/064165
- WO-A2-2018/094190
- US-A- 6 149 962
- US-A1- 2002 187 134
- US-A1- 2017 128 499
- US-A1- 2019 282 632
- SHARMA ANSHUL ET AL: "Role of probiotics in the management of lung cancer and related diseases: An update", JOURNAL OF FUNCTIONAL FOODS, ELSEVIER BV, NL, vol. 40, 22 December 2017 (2017-12-22), pages 625 - 633, XP085357984, ISSN: 1756-4646, DOI: 10.1016/J.JFF.2017.11.050
- VAN DER BEEK CHRISTINA M ET AL: "The prebiotic inulin improves substrate metabolism and promotes short-chain fatty acid production in overweight to obese men", METABOLISM, CLINICAL AND EXPERIMENTAL, W.B. SAUNDERS CO., PHILADELPHIA, PA, US, vol. 87, 25 June 2018 (2018-06-25), pages 25 - 35, XP085493063, ISSN: 0026-0495, DOI: 10.1016/J.METABOL.2018.06.009
- HAN KAI ET AL: "Generation of systemic antitumour immunity via the in situ modulation of the gut microbiome by an orally administered inulin gel", NATURE BIOMEDICAL ENGINEERING, NATURE PUBLISHING GROUP UK, LONDON, vol. 5, no. 11, 24 June 2021 (2021-06-24), pages 1377 - 1388, XP037617183, DOI: 10.1038/S41551-021-00749-2

## Description

### FIELD OF THE INVENTION

This disclosure relates generally to an agent capable of increasing the efficacy of immunotherapies and vaccines. In particular, the present disclosure relates to elevating the richness and diversity of a subject's gut microbiome through administration of an agent (e.g., fiber containing prebiotic agent (e.g., epigallocatechin gallate (EGCG), fucoidan, potato starch, oligofructose and inulin)) (e.g., melatonin) with an immunotherapy or vaccine. The present invention relates to an agent capable of elevating the richness and diversity of the human subject's gut microbiome for use in the treatment of a cancer, the treatment comprising, administering to a human subject suffering from cancer a cancer immunotherapy or vaccine, and said agent capable of elevating the richness and diversity of the human subject's gut microbiome, as defined in the claims.

### BACKGROUND OF THE INVENTION

Cancer immunotherapy is revolutionizing the field of oncology. However, immune checkpoint therapies work only in a subset of patients (typically 10-30%). There is a great need to improve the efficacy of immune checkpoint blockade. In addition, there has been extensive research interest to improve vaccines.

WO2018/094190 (Sanford Burnham Presbys Medical Discovery Inst et al.) relates to the treatment of a cancer in a human comprising orally administering one or more prebiotics, that promote growth of one or more bacteria, and one or more anticancer agents.

WO2018/064165 (Univ Texas) relates to the treatment of cancer by administering butyrate, or a butyrate-producing bacterial population, and an immune checkpoint inhibitor, and further administering a prebiotic or probiotic.

WO2018/222969 (Univ Texas) relates to the increase of efficacy of an immune checkpoint inhibitor for use in treating melanoma comprising administering said immune checkpoint inhibitor in combination with a prebiotic agent.

WO2016/172657 (Kaleido Biosciences Inc.) relates a combination of a glycan and an immune checkpoint inhibitor or cancer vaccine for use in the treatment of a cancer.

Sharma Anshul et al. (Sharma Anshul et al. Journal of Functional Foods,Volume 40, 2018, Pages 625-633) describes an *in vivo* experiment wherein the combined application of an antitumor vaccine with a pro- and/or prebiotic results in a synergestic effect in the therapy for solid sarcoma.

The present invention addresses these needs.

### SUMMARY

References to methods of treatment in this description are to be interpreted as references to the compounds, pharmaceutical compositions and medicaments of the present invention for use in a method of treatment of a human (or animal) body by therapy.

The invention is defined in the accompanying claims. Subjectmatter which is not encompassed by the scope of the claims does not form part of the present claimed invention.

Experiments conducted during the course of developing embodiments for the present invention identified new compounds from FDA approved drugs or diet supplements (e.g., prebiotic agents) that can be ingested to improve the efficacy of immune checkpoint therapies. It was shown that oral formulations of an agent (e.g., fiber containing prebiotic agent (e.g., epigallocatechin gallate (EGCG), fucoidan, potato starch, oligofructose and inulin)) (e.g., melatonin) can alter the gut microbiome so that beneficial bacteria increase in frequency and synergize with immune checkpoint blockers given by the systemic injection. Indeed, it was shown that concurrent administration of prebiotic agents (e.g., inulin) with an immunotherapy (e.g., α-PD-1 therapy) resulted in stronger anti-tumor efficacy, stronger immune response (e.g. AH1-specific CD8⁺ T cells frequency among PBMCs), inhibition of tumor growth, enhanced intratumoral infiltration of T cells, higher IFN-γ expression in splenoic CD8⁺ T cells, and increased abundance of Akkermansia, Lactobacillus, Ruminococcus, Roseburia, and Butyricicoccus within the subject's gut microbiome.

Accordingly, the present disclosure relates generally to an agent capable of increasing the efficacy of immunotherapies and vaccines. In particular, the present disclosure relates to elevating the richness and diversity of a subject's gut microbiome through administration of an agent (e.g., fiber containing prebiotic agent (e.g., epigallocatechin gallate (EGCG), fucoidan, potato starch, oligofructose and inulin)) (e.g., melatonin) with an immunotherapy or vaccine. The present invention relates to an agent capable of elevating the richness and diversity of the human subject's gut microbiome for use in the treatment of a cancer, the treatment comprising, administering to a human subject suffering from cancer a cancer immunotherapy or vaccine, and said agent capable of elevating the richness and diversity of the human subject's gut microbiome, wherein the agent capable of elevating the richness and diversity of the subject's gut microbiome is administered orally, wherein the vaccine is a vaccine for treating or preventing cancer,wherein the cancer is one or more of breast, ovarian, prostate, lung, kidney, gastric, colon, testicular, head and neck, pancreas, brain, melanoma, and other tumors of tissue organs and hematological tumors, such as lymphomas and leukemias, including acute myelogenous leukemia, chronic myelogenous leukemia, chronic lymphocytic leukemia, T cell lymphocytic leukemia, and B cell lymphomas,wherein the administration of the cancer immunotherapy or a vaccine, and the agent capable of elevating the richness and diversity of the subject's gut microbiome, results in one or more of: an increased anti-tumor efficacy of the cancer immunotherapy or vaccine,a stronger immune response (e.g., increased anti-tumor T cell frequency among PBMCs), and enhanced inhibition of tumor growth, wherein the agent capable of elevating the richness and diversity of a subject's gut microbiome is a fiber containing prebiotic agent, wherein the fiber containing prebiotic agent is a gel-based inulin formulation, and wherein the gel-based inulin formulation has an average degree of polymerization at or higher than 20 and at or less than 47.

In certain embodiments, useful for understanding the invention, a method for increasing the efficacy of a cancer immunotherapy or vaccine is provided (e.g., cancer vaccine) (e.g., vaccines against infectious pathogens) through administration of 1) a cancer immunotherapy or vaccine to a subject, and 2) an agent capable of elevating the richness and diversity of the subject's gut microbiome.

In certain embodiments, useful for understanding the invention, a method for inhibiting the ability of a cancer cell to induce immune dysfunction is provided, comprising administration of 1) a cancer immunotherapy or cancer vaccine to a subject, and 2) an agent capable of elevating the richness and diversity of the subject's gut microbiome.

In certain embodiments, useful for understanding the invention, a method for treating or preventing cancer in a subject is provided, comprising administering to the subject 1) a cancer immunotherapy or a cancer vaccine to a subject, and 2) an agent capable of elevating the richness and diversity of the subject's gut microbiome.

According to the disclosure, such methods are not limited to particular manner of administering 1) the cancer immunotherapy or vaccine (e.g., cancer vaccine) (e.g., vaccines against infectious pathogens) to a subject, and 2) the agent capable of elevating the richness and diversity of the subject's gut microbiome. In some embodiments, administration of the agent capable of elevating the richness and diversity of the subject's gut microbiome occurs prior to, concurrent with, and/or after administration of the vaccine (e.g., cancer vaccine) (e.g., vaccines against infectious pathogens) or cancer immunotherapy. In some embodiments, administration of the agent capable of elevating the richness and diversity of the subject's gut microbiome occurs concurrent with administration of the vaccine (e.g., cancer vaccine) (e.g., vaccines against infectious pathogens) or cancer immunotherapy. In some embodiments, administration of the agent capable of elevating the richness and diversity of the subject's gut microbiome occurs prior to administration of the vaccine (e.g., cancer vaccine) (e.g., vaccines against infectious pathogens) or cancer immunotherapy. In some embodiments, administration of the agent capable of elevating the richness and diversity of the subject's gut microbiome occurs prior to and concurrent with administration of the vaccine (e.g., cancer vaccine) (e.g., vaccines against infectious pathogens) or cancer immunotherapy.

Such methods are not limited to a particular type of subject. In some embodiments, the subject is a human subject.

According to the disclosure, such methods are not limited to particular type or kind of agent capable of elevating the richness and diversity of a subject's gut microbiome. In some embodiments, the agent is a fiber containing prebiotic agent. In some embodiments, the fiber containing prebiotic agent is selected from epigallocatechin gallate (EGCG), fucoidan, potato starch, oligofructose and inulin.

According to the invention, the fiber containing prebiotic agent is a gel-based inulin formulation as defined in the claims. Inulin is a polysaccharide belonging to the fructan group. It consists of a beta-2-1-linked chain of fructose molecules, this chain having at its end an alpha-D-glucose unit at the reducing end. Inulin occurs in economically recoverable amounts in various plants such as, for example, chicory roots and dahlia tubers. In addition, inulin has been found for example in Jerusalem artichokes and artichokes. The average chain lengths of the various inulins and their physico-chemical properties differ from plant species to plant species. According to the invention, the agent is a gel-based inulin formulation having an average degree of polymerization at or higher than 20 and at or less than 47. In some embodiments, the agent is a gel-based inulin formulation having an average degree of polymerization at approximately 28 (e.g., 23, 24, 25, 26, 27, 28, 29, 30, 31, 32, 33) (see, Example XIII showing surprising anti-tumor efficacy with a gel-based inulin formulation having an average degree of polymerization at approximately 28).

In some embodiments, the gel-based inulin formluation comprises one or more prebiotic compounds selected from a fructo-oligosaccharide, a short-chain fructo-oligosaccharide, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber. In some embodiments, the agent is melatonin.

Such methods are not limited to a particular type or kind of caner immunotherapy. In some embodiments, the cancer immunotherapy comprises one or more immune checkpoint inhibitor (ICI) inhibitors. In some embodiments, the one or more ICI inhibitors are capable of binding to, blocking, and/or inhibit the activity of one or more of CTLA-4, PDL1, PDL2, PD1, BTLA, HVEM, TIM3, GAL9, LAG3, VISTA, KIR, 2B4, CD160 and CGEN-15049. In some embodiments, the one or more ICI inhibitors are selected from Tremelimumab (CTLA-4 blocking antibody), anti-OX40, PD-L1 monoclonal Antibody (Anti-B7-H1; MEDI4736), MK-3475 (PD-1 blocker), Nivolumab (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and Yervoy/ipilimumab (anti-CTLA-4 checkpoint inhibitor).

In general, the cancer is any type of cancer responsive to cancer immunotherapy or cancer vaccine treatment. According to the invention, the cancer is one or more of breast, ovarian, prostate, lung, kidney, gastric, colon, testicular, head and neck, pancreas, brain, melanoma, and other tumors of tissue organs and hematological tumors, such as lymphomas and leukemias, including acute myelogenous leukemia, chronic myelogenous leukemia, chronic lymphocytic leukemia, T cell lymphocytic leukemia, and B cell lymphomas.

In some embodiments, such methods further comprise administering to the subject one or more chemotherapeutic agents selected from the group consisting of an alkylating agent, an antimetabolite, an anthracycline, an antitumor antibiotic, a monoclonal antibody, a platinum agent, a plant alkaloid, a topoisomerase inhibitor, a vinca alkaloid, a taxane, and an epipodophyllotoxin.

According to the disclosure, such methods are not limited to a particular manner of administration of the agent capable of elevating the richness and diversity of the subject's gut microbiome. According to the invention, the agent is administered orally. In some embodiments, the agent is administered by oral gavage.

In some embodiments for such methods, the agent capable of elevating the richness and diversity of the subject's gut microbiome results in increased relative abundance of Akkermansia, Lactobacillus, Ruminococcus, Roseburia, and Butyricicoccus within the gut microbiome of the subject.

In some embodiments for such methods, administration of 1) a cancer immunotherapy or vaccine (e.g., cancer vaccine) (e.g., vaccines against infectious pathogens) to a subject, and 2) administration of an agent capable of elevating the richness and diversity of the subject's gut microbiome, results in one or more of an increased anti-tumor efficacy of the cancer immunotherapy or vaccine (e.g., cancer vaccine) (e.g., vaccines against infectious pathogens), a stronger immune response (e.g., increased anti-tumor T cell frequency among PBMCs), and enhanced inhibition of tumor growth.

The present invention provides a composition comprising a gel-based inulin formulation which has an average degree of polymerization at or higher than 20 and at or less than 47. In some embodiments, the gel-based inulin formulation has an average degree of polymerization at approximately 28 (e.g., 23, 24, 25, 26, 27, 28, 29, 30, 31, 32, 33).

In some embodiments, the gel-based inulin formluation comprises one or more prebiotic compounds selected from a fructo-oligosaccharide, a short-chain fructo-oligosaccharide, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a resistant potato starch, guar gum, bean gum, gelatin,glycerol, a polydextrose, a D-tagatose, an acacia fiber, carob, an oat, and a citrus fiber.

In certain embodiments, useful for understanding the invention, methods for treating or preventing a condition characterized with dysregulated gut microbiome activity are provided, comprising administering to the subject an agent capable of elevating the richness and diversity of the subject's gut microbiome.

In some embodiments, the subject is a human subject.

In some embodiments, the agent capable of elevating the richness and diversity of a subject's gut microbiome is a fiber containing prebiotic agent. In some embodiments of the disclosure, the fiber containing prebiotic agent is selected from epigallocatechin gallate (EGCG), fucoidan, potato starch, oligofructose and inulin. In some embodiments, the fiber containing prebiotic agent is a gel-based inulin formulation. In some embodiments, the gel-based inulin formulation has an average degree of polymerization at or higher than 20 and at or less than 47. In some embodiments, the gel-based inulin formulation has an average degree of polymerization at approximately 28 (e.g., 23, 24, 25, 26, 27, 28, 29, 30, 31, 32, 33). In some embodiments, the gel-based inulin formluation comprises one or more prebiotic compounds selected from a fructo-oligosaccharide, a short-chain fructo-oligosaccharide, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber. In some embodiments of the disclosure, the agent capable of elevating the richness and diversity of a subject's gut microbiome is melatonin.

According to the invention, the agent capable of elevating the richness and diversity of the subject's gut microbiome is administered orally.

In some embodiments, administration of the agent capable of elevating the richness and diversity of the subject's gut microbiome results in increased relative abundance of Akkermansia, Lactobacillus, Ruminococcus, Roseburia, and Butyricicoccus within the gut microbiome of the subject.

In some embodiments of the disclosure, the condition characterized with dysregulated gut microbiome activity is an autoimmune disease, a neurological disorder, diabetes, and/or obesity.

In some embodiments of the disclosure, the condition characterized with dysregulated gut microbiome activity is selected from rheumatoid arthritis, multiple sclerosis diabetes (e.g., type 1 diabetes mellitus), autoimmune diseases of the thyroid (e.g., Hashimoto's thyroiditis, Graves' disease), thyroid-associated ophthalmopathy and dermopathy, hypoparathyroidism, Addison's disease, premature ovarian failure, autoimmune hypophysitis, pituitary autoimmune disease, immunogastritis, pernicious angemis, celiac disease, vitiligo, myasthenia gravis, pemphigus vulgaris and variants, bullous pemphigoid, dermatitis herpetiformis Duhring, epidermolysis bullosa acquisita, systemic sclerosis, mixed connective tissue disease, Sjogren's syndrome, systemic lupus erythematosus, Goodpasture's syndrome, rheumatic heart disease, autoimmune polyglandular syndrome type 1, Aicardi-Goutières syndrome, Acute pancreatitis Age-dependent macular degeneration, Alcoholic liver disease, Liver fibrosis, Metastasis, Myocardial infarction, Nonalcoholic steatohepatitis (NASH), Parkinson's disease, Polyarthritis/fetal and neonatal anemia, Sepsis, and inflammatory bowel disease.

In some embodiments of the disclosure, the method further comprises administering to the subject one or more of the following additional therapeutic agents: disease-modifying antirheumatic drugs (e.g., leflunomide, methotrexate, sulfasalazine, hydroxychloroquine), biologic agents (e.g., rituximab, infliximab, etanercept, adalimumab, golimumab), nonsteroidal anti-inflammatory drugs (e.g., ibuprofen, celecoxib, ketoprofen, naproxen, piroxicam, diclofenac), analgesics (e.g., acetaminophen, tramadol), immunomodulators (e.g., anakinra, abatacept), glucocorticoids (e.g., prednisone, methylprednisone), TNF-α inhibitors (e.g., adalimumab, certolizumab pegol, etanercept, golimumab, infliximab), IL-1 inhibitors, and metalloprotease inhibitors. In some embodiments, the therapeutic agents include, but are not limited to, infliximab, adalimumab, etanercept, parenteral gold or oral gold.

In certain embodiments, useful for understanding the invention, a method for increasing the efficacy of a vaccine is provided through administration of 1) a vaccine to a subject, and 2) administration of an agent capable of elevating the richness and diversity of the subject's gut microbiome.

In some embodiments, administration of the agent capable of elevating the richness and diversity of the subject's gut microbiome occurs prior to, concurrent with, and/or after administration of the vaccine.

In some embodiments, administration of the agent capable of elevating the richness and diversity of the subject's gut microbiome occurs concurrent with administration of the vaccine; or administration of the agent capable of elevating the richness and diversity of the subject's gut microbiome occurs prior to administration of the vaccine. In some embodiments, wherein administration of the agent capable of elevating the richness and diversity of the subject's gut microbiome occurs prior to and concurrent with administration of the vaccine.

In some embodiments, the vaccine is a vaccine for treating cancer, and/or a vaccine for treating and/or protecting from infectious pathogens. According to the invention, the vaccine is a vaccine for treating cancer.

In some embodiments, the subject is a human subject.

In some embodiments, the agent capable of elevating the richness and diversity of a subject's gut microbiome is a fiber containing prebiotic agent. In some embodiments of the disclosure, the fiber containing prebiotic agent is selected from epigallocatechin gallate (EGCG), fucoidan, potato starch, oligofructose and inulin. According to the invention, the fiber containing prebiotic agent is a gel-based inulin formulation which has an average degree of polymerization at or higher than 20 and at or less than 47. In some embodiments, the gel-based inulin formulation has an average degree of polymerization at approximately 28 (e.g., 23, 24, 25, 26, 27, 28, 29, 30, 31, 32, 33). In some embodiments, the gel-based inulin formluation comprises one or more prebiotic compounds selected from a fructo-oligosaccharide, a short-chain fructo-oligosaccharide, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber. In some embodiments, the agent capable of elevating the richness and diversity of a subject's gut microbiome is melatonin.

According to the invention, the agent capable of elevating the richness and diversity of the subject's gut microbiome is administered orally.

In some embodiments, administration of the agent capable of elevating the richness and diversity of the subject's gut microbiome results in increased relative abundance of Akkermansia, Lactobacillus, Ruminococcus, Roseburia, and Butyricicoccus within the gut microbiome of the subject.

The present disclosure also provides kits comprising an agent capable of elevating the richness and diversity of the subject's gut microbiome (e.g., a fiber based pre-biotic), and one or more of a vaccine (e.g., cancer vaccine) (e.g., vaccines against infectious pathogens), and a cancer immunotherapy (e.g., an ICI inhibitor). The kits may optionally contain other therapeutic agents.

In certain embodiments of the disclosure, the present disclosure provides compositions comprising a prebiotic formulation associated with (e.g., complexed, conjugated, encapsulated, absorbed, adsorbed, admixed) bacteria.

In some embodiments of the disclosure, the prebiotic formulation comprises one or more prebiotic compounds selected from a fructo-oligosaccharide, a short-chain fructo-oligosaccharide, inulin, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber.

In some embodiments of the disclosure, the prebiotic compound is gel-based. In some embodiments, the prebiotic compound is gel-based inulin.

In some embodiments of the disclosure, the bacteria is E. coli. In some embodiments, the bacteria is able to alter the gut microbiome of a subject upon administration to the subject.

In some embodiments, the composition is configured for oral administration to a subject.

In certain embodiments, useful for understanding the invention, methods for colonic delivery of bacteria to a subject are provided, comprising administering to a subject such a comprising a prebiotic formulation associated with (e.g., complexed, conjugated, encapsulated, absorbed, adsorbed, admixed) bacteria (e.g., gel-based inulin).

In certain embodiments, useful for understanding the invention, compositions are provided comprising a prebiotic formulation associated with (e.g., complexed, conjugated, encapsulated, absorbed, adsorbed, admixed) one or more probiotic cells. In certain embodiments, the present disclosure provides methods for increasing the growth of probiotic organisms in the digestive system of a subject, comprising administering to the subject (e.g., mammalian subject; human subject) such a composition.

In some embodiments of the disclosure, the prebiotic formulation comprises one or more prebiotic compounds selected from a fructo-oligosaccharide, a short-chain fructo-oligosaccharide, inulin, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber.

In some embodiments, the prebiotic compound is gel-based. In some embodiments, the prebiotic compound is gel-based inulin.

In some embodiments, the one or more probiotic cells are able to alter the gut microbiome of a subject upon administration to the subject.

In some embodiments, the composition is configured for oral administration to a subject.

In some embodiments of the disclosure, the one or more probiotic cells comprise beneficial bacteria. In some embodiments, the beneficial bacteria comprises one or more of: Saccharomyces cereviseae, Bacillus coagulans, Bacillus licheniformis, Bacillus subtilis, Bifidobacterium angulatum, Bifidobacterium animalis, Bifidobacterium bifidum, Bifidobacterium breve, Bifidobacterium infantis, Bifidobacterium lactis, Bifidobacterium longum, Enterococcus faecium, Enterococcus faecalis, Lactobacillus acidophilus, Lactobacillus amylovorus, Lactobacillus alimentarius, Lactobacillus bulgaricus, Lactobacillus casei subsp. casei, Lactobacillus casei Shirota, Lactobacillus curvatus, Lactobacillus delbrueckii subsp. lactis, Lactobacillus fermentum, Lactobacillus farciminus, Lactobacillus gasseri, Lactobacillus helveticus, Lactobacillus johnsonii, Lactobacillus lacti, Lactobacillus paracasei, Lactobacillus pentosaceus, Lactobacillus plantarum, Lactobacillus reuteri, Lactobacillus rhamnosus (Lactobacillus GG), Lactobacillus sake, Lactobacillus salivarius, Lactococcus lactis, Lactobacillus thermotolerans, Lactobacillus mucosae, Micrococcus varians, Pediococcus acidilactici, Pediococcus pentosaceus, Pediococcus acidilactici, Pediococcus halophilus, Streptococcus faecalis, Streptococcus thermophilus, Staphylococcus carnosus, and Staphylococcus xylosus.

In some embodiments, the composition is a sugar-coated tablet, gel capsule, gel, emulsion, tablet, wafer capsule, hydrogel, nanofiber gel, electrospun fiber, food bar, confectionery, fermented milk, fermented cheese, chewing gum, powder or toothpaste.

In certain embodiments, the present disclosure provides a composition comprising a gel-based prebiotic formulation. In some embodiments, the composition is formulated for oral ingestion. In some embodiments, the composition is a sugar-coated tablet, gel capsule, gel, emulsion, tablet, wafer capsule, hydrogel, nanofiber gel, electrospun fiber, food bar, confectionery, fermented milk, fermented cheese, chewing gum, powder or toothpaste.

In some embodiments of the disclosure, the prebiotic formulation comprises one or more prebiotic compounds selected from a fructo-oligosaccharide, a short-chain fructo-oligosaccharide, inulin, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber.

In some embodiments of the disclosure, the gel-based prebiotic formulation is associated with one or more probiotic organisms. In some embodiments, the one or more probiotic organisms are chosen from Lactobacillus species and Bifidobacterium species. In some embodiments, the gel-based prebiotic formulation is associated with one or more bacteriophages specific to Bordetella, Borrelia, Brucella, Campylobacter, Chlamydia and Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema, Vibrio and Yersinia. In some embodiments, the one or more probiotic organisms are selected from L. acidophilus, L. amylovorus, L. brevis, L. casei, L. casei subsp. rhamnosus (Lactobacillus GG), L. caucasicus, L. crispatus, L. delbrueckii subsp. bulgaricus (L. bulgaricus), L. fermentum (L. fermenti), L. gasseri, L. helveticus, L. johnsonii, L. lactis, L. leichmannii, L. paracasei, L. plantarum, L. reuteri, or L. rhamnosus.

### BRIEF DESCRIPTION OF THE DRAWINGS

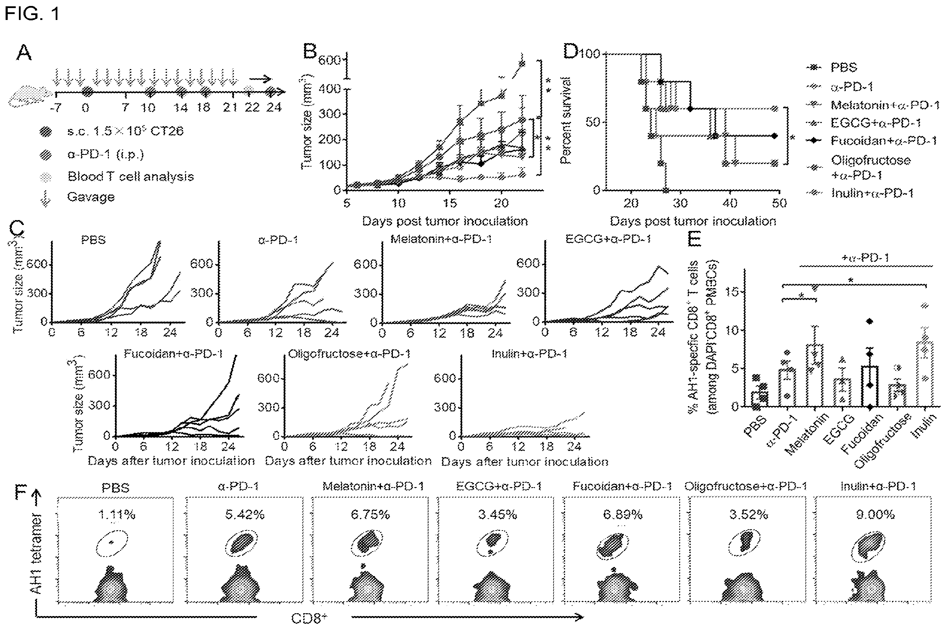

FIG. 1A-F: In vivo screening of the candidate materials to prophylactically improve the anti-tumor efficacy of α-PD-1. (A) Mice were prophylactically gavaged various samples on day - 7 for three times. Then 1.5 × 10⁵ CT26 cells were inoculated in BALB/c mice subcutaneously (s.c.) on day 0. Mice were gavaged three times again in the first week post tumor inoculation and 5 times per week from day 7. α-PD-1 was i.p. injected on days 10, 14, 18 and 24 at 100 µg/dose. (B) Individual tumor growth curves and (C) average tumor growth curves for mice gavaged with various compounds. (D) Survival rate curves of various groups. (E) The average frequency of AH1-specific CD8⁺ T cells in peripheral blood on day 22. (F) The representative scatter plots were measured via flow cytometry. Data represent mean ± SEM. **p*<0.05, ***p*<0.01, analyzed by two-way ANOVA (B), or t-test (E), log-rank (Mantel-Cox) test (D).

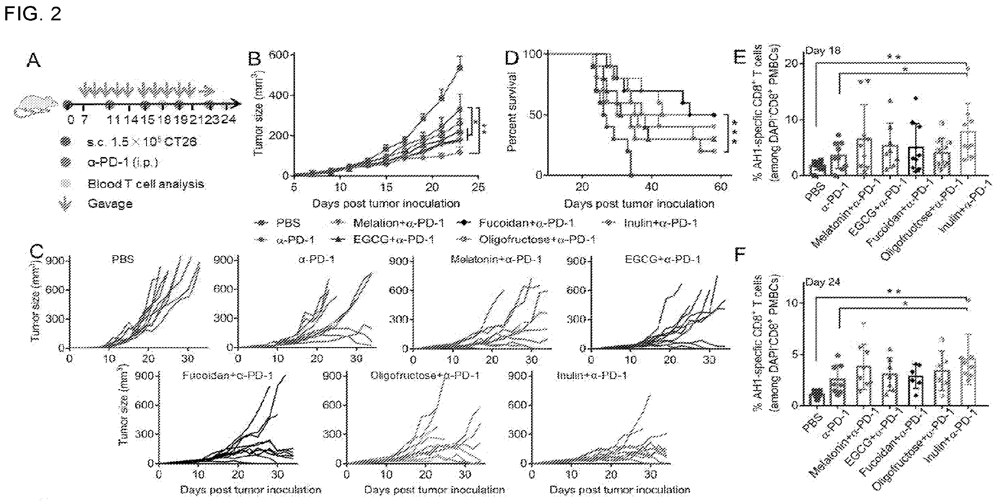

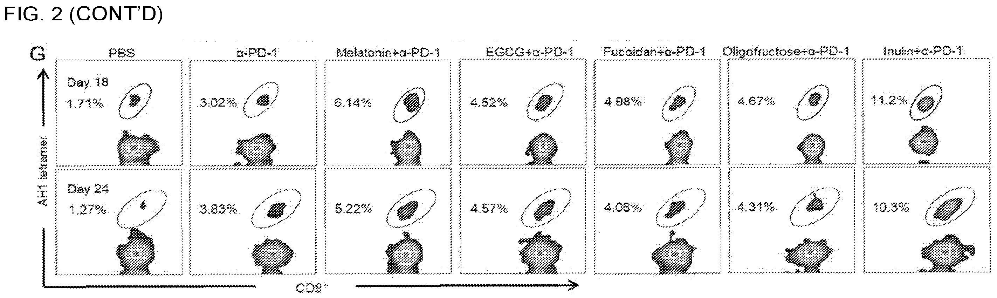

FIG. 2A-G: In vivo screening of the candidate materials to improve the anti-tumor efficacy of α-PD-1. (A) 1.5 × 10⁵ CT26 cells were inoculated in BALB/c mice subcutaneously (sc) on day 0. Mice were gavaged from day 7 (5 times per week). On days 11, 15, 19 and 23, α-PD-1 was injected intraperitoneally (ip) at 100 µg per dose. (B) The average tumor growth curves for mice gavaged with various compounds. Data represent mean ± SEM. (C) Individual tumor growth curves and (D) survival rate curves of various groups. On day 18 and 24, peripheral blood was collected. The average frequency of AH1-specific CD8⁺ T cells at (E) day 18, (F) day 24 and (G) the representative scatter plots were measured via flow cytometry. Data represent mean ± SD. All the data are from 2-3 independent experiments; n = 10 (B, C, D), n = 7-10 (E, F) **p*<0.05, ***p*<0.01, analysed by two-way ANOVA (B), one-way ANOVA (E, F) or log-rank (Mantel-Cox) test.

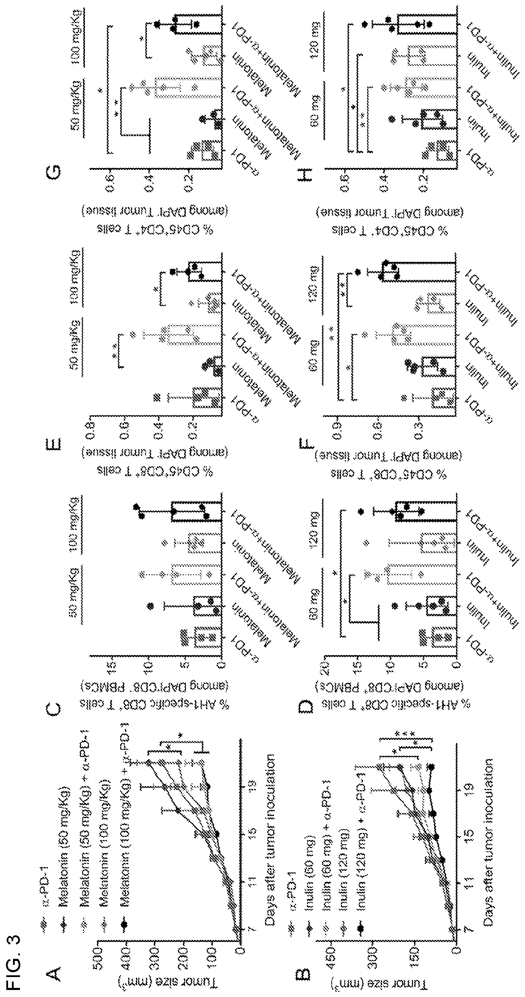

FIG. 3A-H: In vivo assessment of the dosage effect of melatonin and inulin combined with α-PD-1. 1.5 × 10⁵ CT26 cells were s.c. inoculated in BALB/c mice on day 0. Mice were gavaged from day 7 (5 times per week). On days 11, 15, 19 and 23, α-PD-1 was i.p. injected at 100 µg per dose. The average tumor growth curves for mice gavaged with (A) melatonin and (B) inulin at different dosages. The average frequency of AH1-specific CD8⁺ T cells on day 18 after treatment with (C) melatonin and (D) inulin at different dosages. On day 24, the percentage of CD45⁺CD8⁺ T cells in whole tumor tissue after treatment with (E) melatonin and (F) inulin at different dosages. On day 24, the percentage of CD45⁺CD4⁺ T cells in all cells after treatment with (G) melatonin and (H) inulin at different dosages. Data represent mean ± SEM (n=5). **p*<0.05, ***p*<0.01, ****p<0.001,* analyzed by two-way ANOVA (A, B) or two tails, unpaired t-test (D), one-way ANOVA (E, F, G, H).

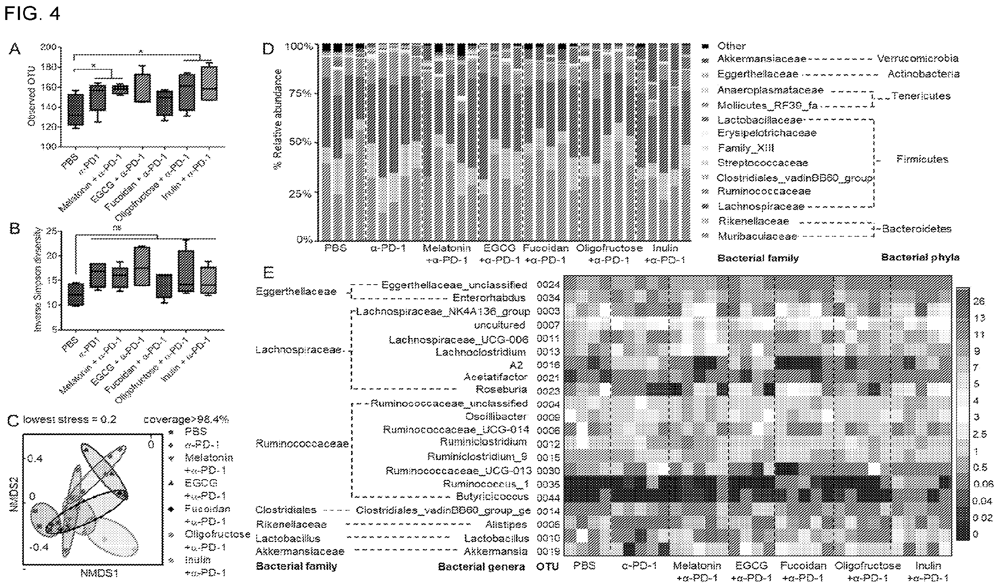

FIG. 4A-H: Responses of the diversity, richness, and structure of the gut microbiota. 1.5 × 10⁵ CT26 cells were inoculated in BALB/c mice subcutaneously and gavaged from day 7 (5 times per week). α-PD-1 (100 µg per dose) was ip injected on days 11, 15, 19 and 23. The fecal pellets were collected on day 21 for 16s RNA analysis. (A) OTU number, (B) inverse Simposon diversity and (C) nonmetric multidimensional scaling (NMDS) score plot (based on Bray-Curtis) of gut microbiota in various groups. (D) Relative abundances of the gut microbiota at family level and (I) genus level. (F) The relative abundances of lactobacillus, akkermansia, roseburia and Ruminococcus_1 in various groups. (G) The relationship of between the tumor sizes and the relative abundances of lactobacillus, akkermansia, roseburia and Ruminococcus_1. (H) The heatmap of SCFAs (lactate, propionate and butyrate) levels of various groups in fecal pellets. Data represent mean ± SEM. **p*<0.05, ***p<0.01,* ****p<0.0001, analysed by one-way ANOVA (A, B, F).

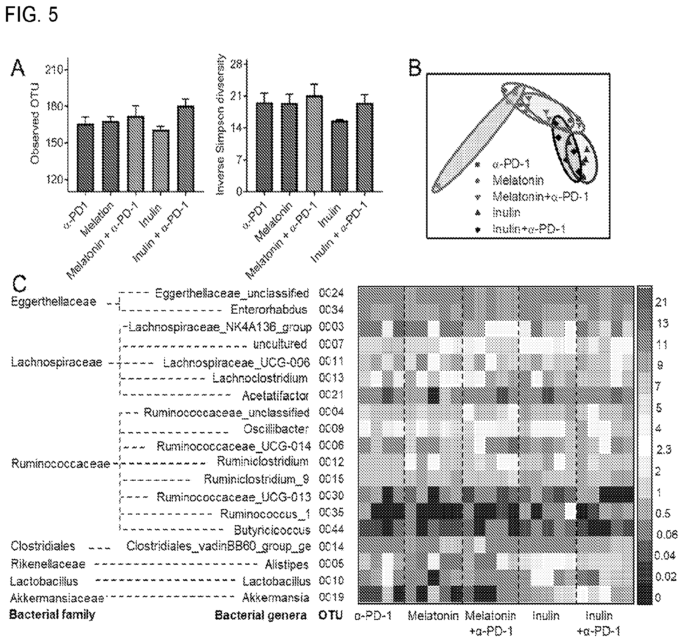

FIG. 5A-C: Gut microbiota analysis. 1.5 × 10⁵ CT26 cells were inoculated in BALB/c mice subcutaneously and gavaged from day 7 (5 times per week). α-PD-1 (100 µg per dose) was ip injected on days 11, 15, 19 and 23. The fecal pellets were collected on day 21 for 16s RNA analysis. (A) OTU number, (B) NMDS score plot of gut microbiota in various groups. (C) Relative abundances of the gut microbiota at genus level. Data represent mean ± SEM.

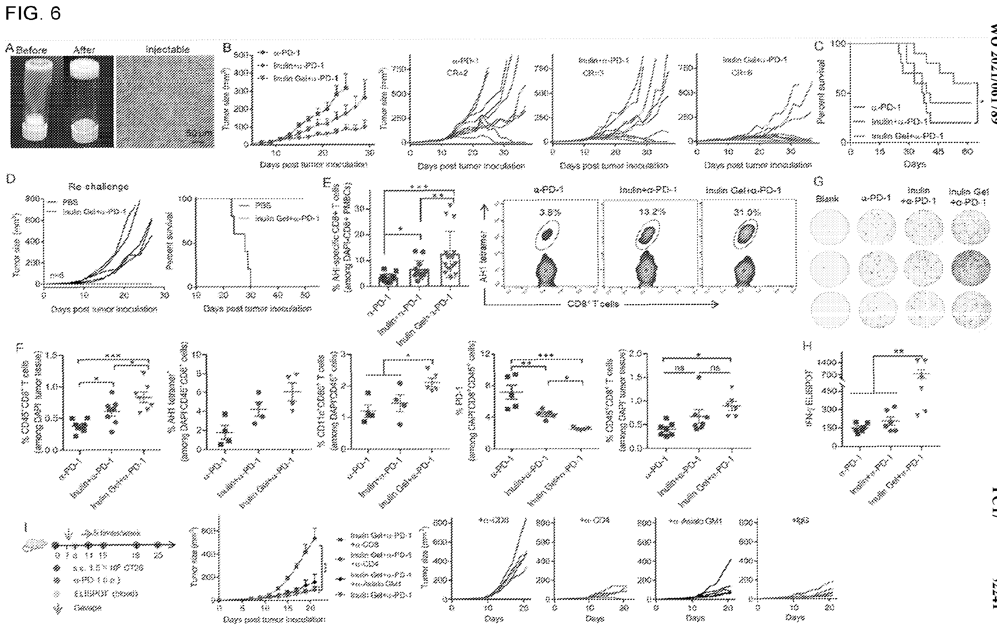

FIG. 6A-I: Inulin gel further enhance the therapeutic efficacy of α-PD-1. (A) Image of inulin gel and the SEM image. (B) The average tumor growth curves and individual tumor growth curve for mice gavaged with inulin or inulin gel (60mg/dosage). 1.5 × 10⁵ CT26 cells were inoculated in BALB/c mice subcutaneously. Gavage started from day 7 (5 times per week) and 100 µg of α-PD-1 was ip injected on days 11, 15, 19, 23 and 27. (C) The survival rate curves of various groups. (D) Tumor-eradicated mice in inulin gel combined with α-PD-1 group was injected with 1.5 × 10⁵ CT26 cells and tumor size was monitored. PBS was used as a control. (E) The average frequency and representative scatter plots of AH1-specific CD8⁺ T cells at day 23. (F) Tumor microenvironment analysis: the percentage of CD45⁺CD8⁺ T cells in whole tumor; AH1-tetramer positive in DAPI⁻CD45⁺CD8⁺ T cells; matured DC cells (CD11c⁺CD86⁺ T cells in DAPI⁻CD45⁺ cells) and CD45⁺CD4⁺ T cells in whole tumor. (G, H) On day 23, spleen were obtained for IFN-γ ELISPOT test. (I) CT26 tumor-bearing BALB/c mice were injected with 200 µg of antibodies targeted against CD8⁺ T cells (αCD8), CD4⁺ T cells (αCD4) and NK cells (αAsialo GM1) on day 8, 11 and 16. α-PD-1 injection as well as sample gavage employed the same regime mentioned above. Shown are the average and individual tumor growth curves for tumor-bearing mice. IgG antibody was used as a control group. Data represent mean ± SEM. All the data are from 2-3 independent experiments; n = 10 (B, C), n = 5 (D, I), n = 10-15 (E), n = 4-7 (G, F), **p*<0.05, ***p*<0.01, ****p*<0.001, analysed by one-way ANOVA (E, F), two-way ANOVA (B, I), or log-rank (Mantel-Cox) test (C).

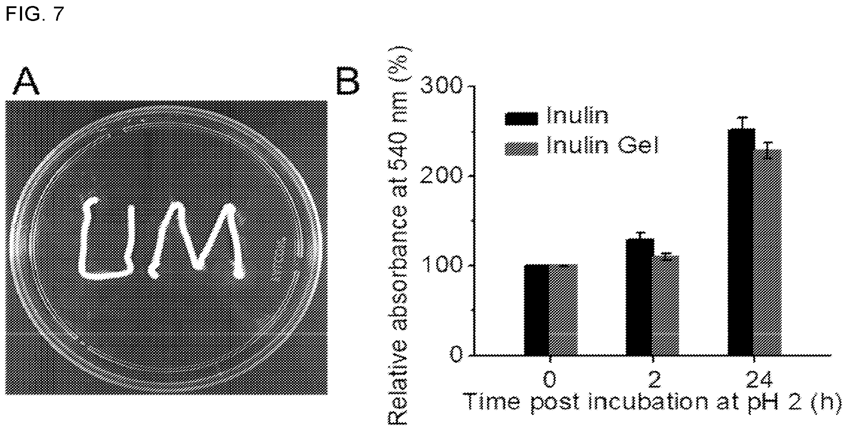

FIG. 7A-B: (A) Inulin gel was injected through syringe (1 mL) into DI water. Image of inulin gel shows the stability in water. (B) The stability of inulin gel when inulin gel was incubated under a strong acid environment (pH 2) for 2 and 24 h, respectively. DNS reagent was used at probe and the absorbance was measured to verify the content of reduced sugar. Inulin gel without acidic treatment was used as a standard and inulin was used as a control.

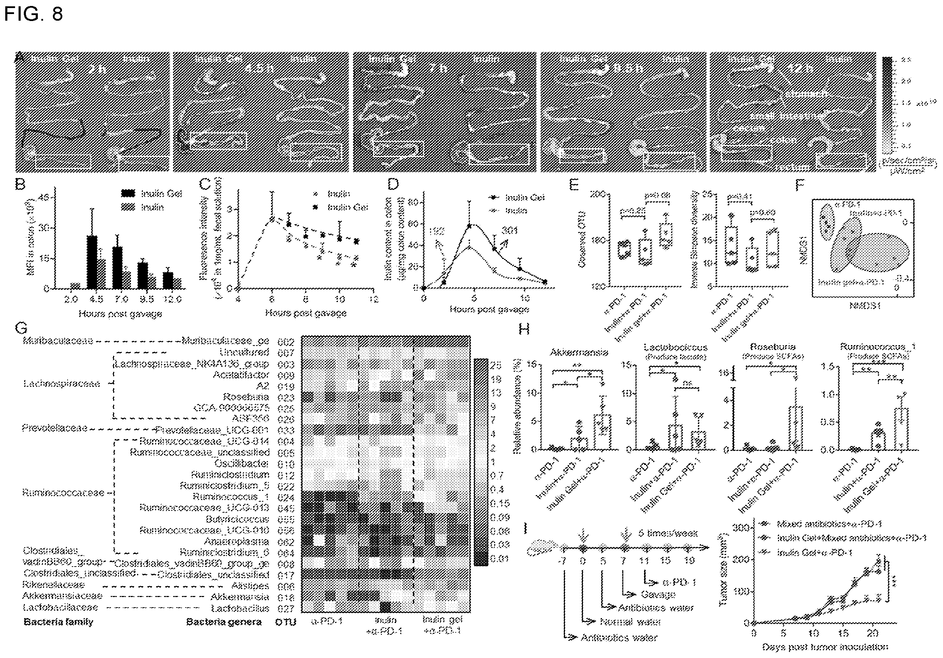

FIG. 8A-I: Inulin gel exhibited prolonged colon retention. (A) IVIS imaging of intestinal retention of inulin gel at different time point (white box indicated colon region) and (B) the corresponding mean fluorescence intensity in colon region. Inulin was label with FITC. (C) The fluorescence intensity of inulin-FITC in fecal pellet at different time points post gavage. (D) The inulin content in colon digesta determined by commercial inulin kit. Arrow indicated the area under curve (AUC) value of inulin. The fecal pellets were collected on day 21 and the 16s RNA analysis was performed. (E) OTU number, inverse Simposon diversity and (F) NMDS score plot (based on Bray-Curtis) of gut microbiota in various groups. (G) Relative abundances of the gut microbiota genus level and (H). (I) Naive mice were fed with broad-spectrum antibiotics (ampicillin + colistin + streptomycin) for one week. Then 1.5× 105 CT26 cells were inoculated in BALB/c mice subcutaneously, mice were fed with normal drinking water for 5 days, followed with continuous broad-spectrum antibiotics treatment. α-PD-1 injection as well as sample gavage employed the same regime mentioned above. Shown are the average tumor growth curves. Data represent mean ± SEM. Data are from 2-3 independent experiments (A-D); **p*<0.05, ***p*<0.01, ****p*<0.001, *****p*<0.0001, analysed by one-way ANOVA (C, E, H) or two-way ANOVA (I)

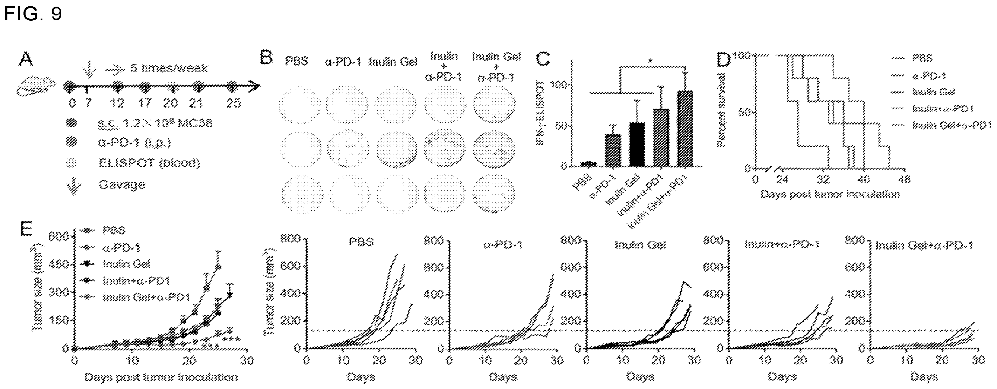

FIG. 9A-E: (A) MC38 tumor model was established on C57BL/6 mice and received α-PD-1 injection and sample gavage. (B) On day 20, PBMCs were obtained for IFN-γ ELISPOT assay. (D) The survival rate curves and (E) average and individual tumor growth curves of various groups. Data represent mean ± SEM. **p*<0.05, ***p*<0.01, ****p*<0.001, *****p*<0.0001, analyzed by one-way ANOVA (A, B, G).

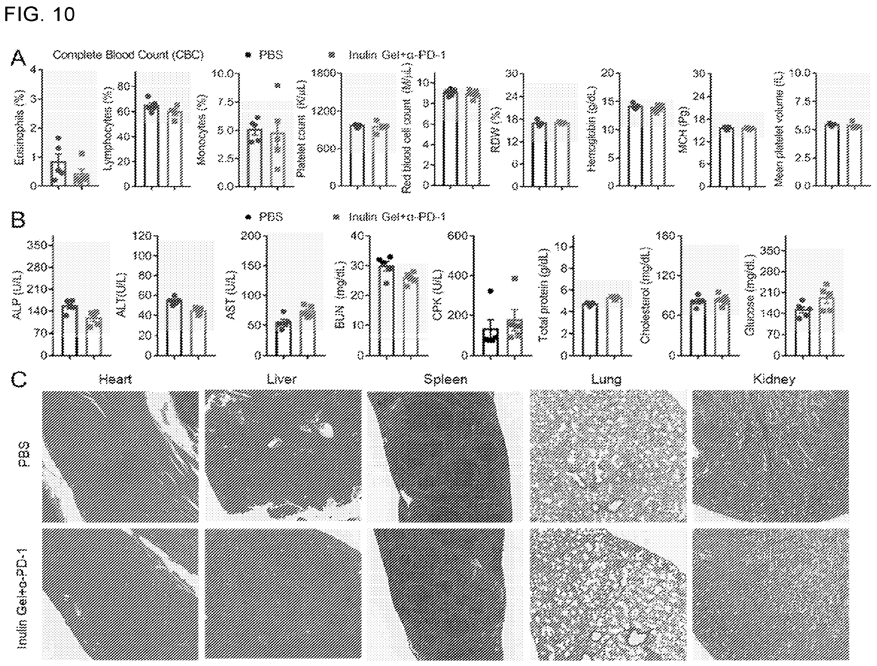

FIG. 10A-C: Biosafety study of inulin gel combined with α-PD-1. (A) The complete blood count panel including eosinophils, lymphocytes, monocytes, platelet count, red blood cell count, RDW, hemoglobin, MCH and mean platelet volume. (B) The biochemistry panel including ALP, ALT, AST, BUN, CPK, total protein, cholesterol and glucose. (C) H&E staining of heart, liver, spleen, lung, kidneys after treatment of inulin gel combined with α-PD-1 on day 29.

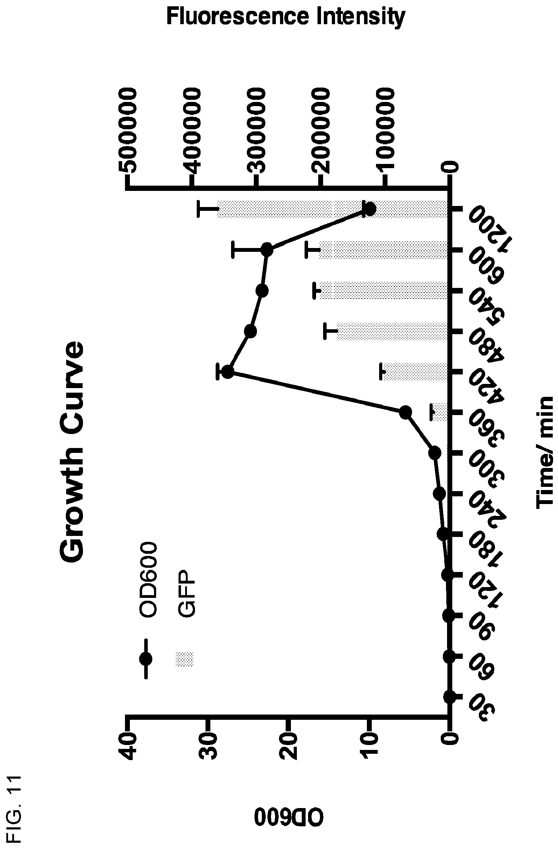

FIG. 11: E. coli growth curve and GFP expression. E. coli was culture in 250 mL Erlenmeyer flask. Culture was sampled at different intervals. OD₆₀₀ and relative fluorescence intensity were measured.

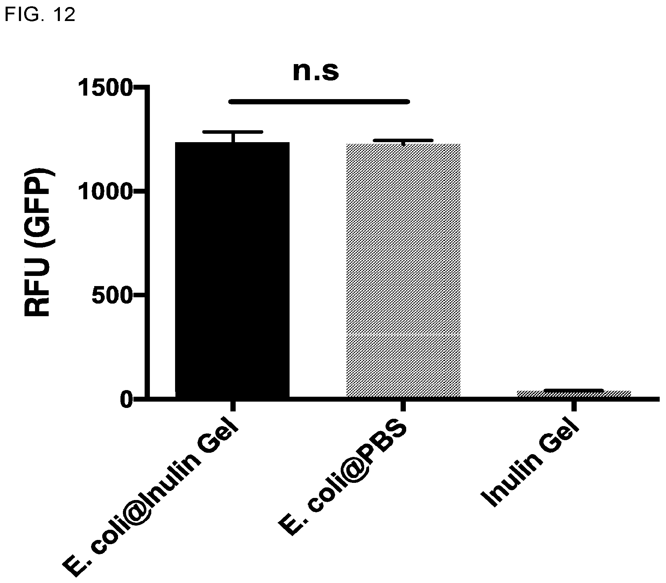

FIG. 12: Cell Viability determined by fluorescence Intensity after 24hrs incubation in PBS and encapsulation in inulin gel respectively.

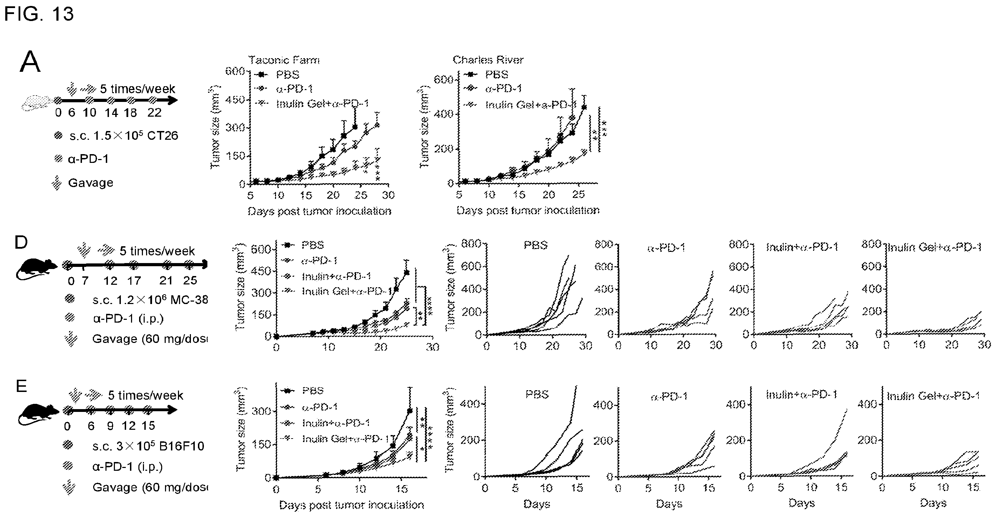

FIG. 13A-E: Validation of the anti-tumor efficacy of inulin gel plus α-PD-1 combotherapy in other models. (A) Treatment regimen of CT26 tumor-bearing BALB/c mice from Taconic Farm or Charles River. Shown are the tumor growth among BALB/c mice from (B) Taconic Farm or (C) Charles River. D-E) C57BL/6 mice bearing (D) MC-38 colon carcinoma and (E) B16F10 melanoma were treated as shown (inulin: 60 mg/dose), and tumor growth was monitored.

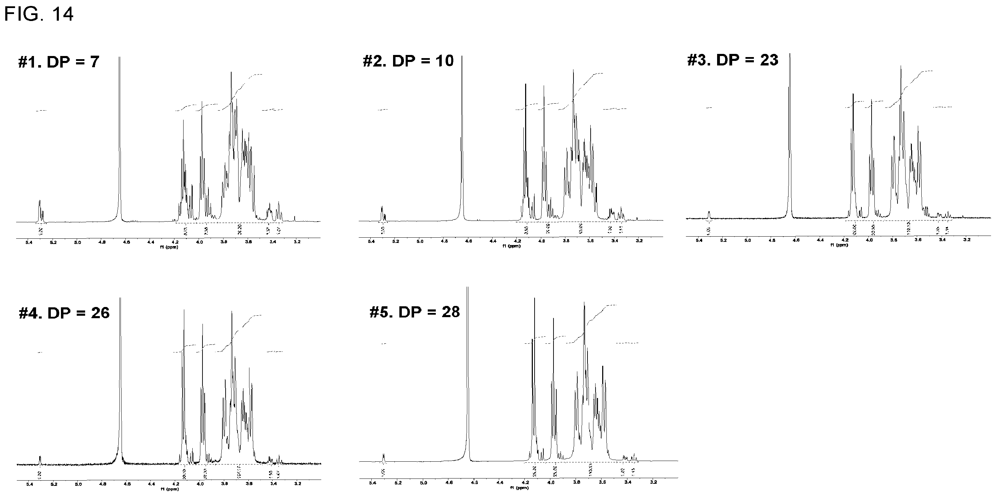

FIG. 14: ¹H NMR spectra of various inulin samples.

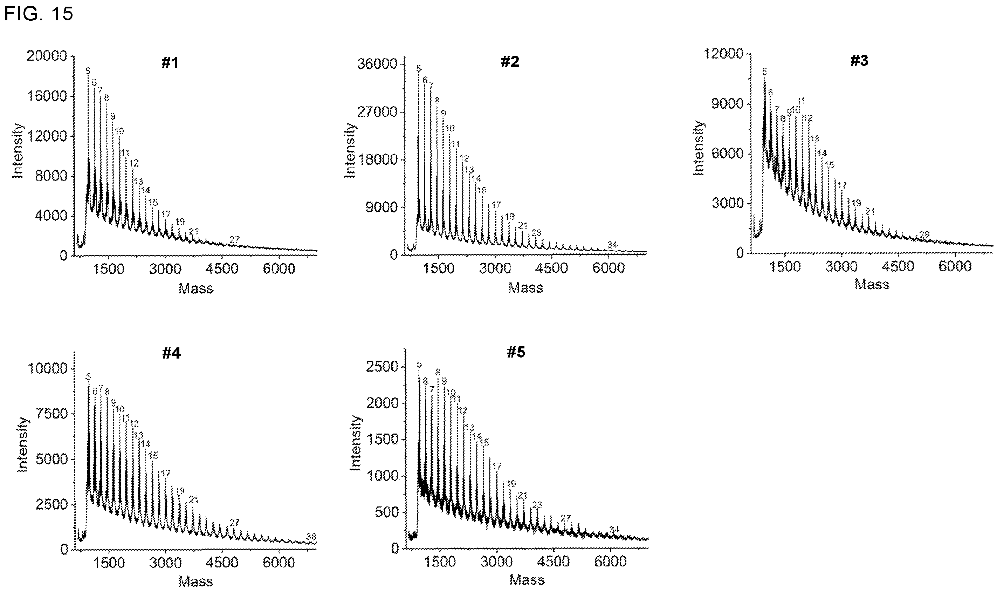

FIG. 15: MALDI-TOF mass spectrum of inulin samples.

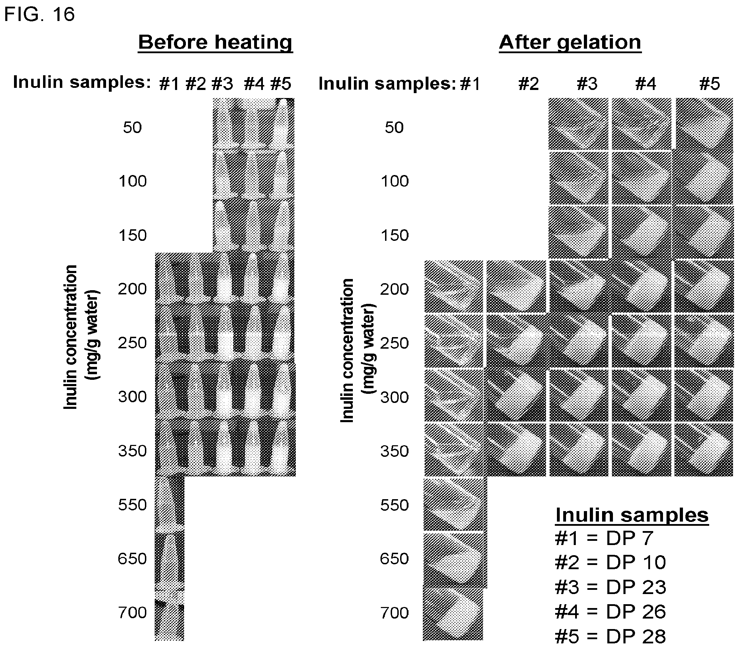

FIG. 16: Impact of inulin concentration and average DP on inulin gel formation. Various inulin gel formulations were formed using inulin of different DPs. Inulin with the average DP of 7, 10, 23, 26, and 28 were dissolved in water in various concentrations. After inducing gelation via heating and cooling, inulin gel formation was observed with high inulin concentration and high DPs.

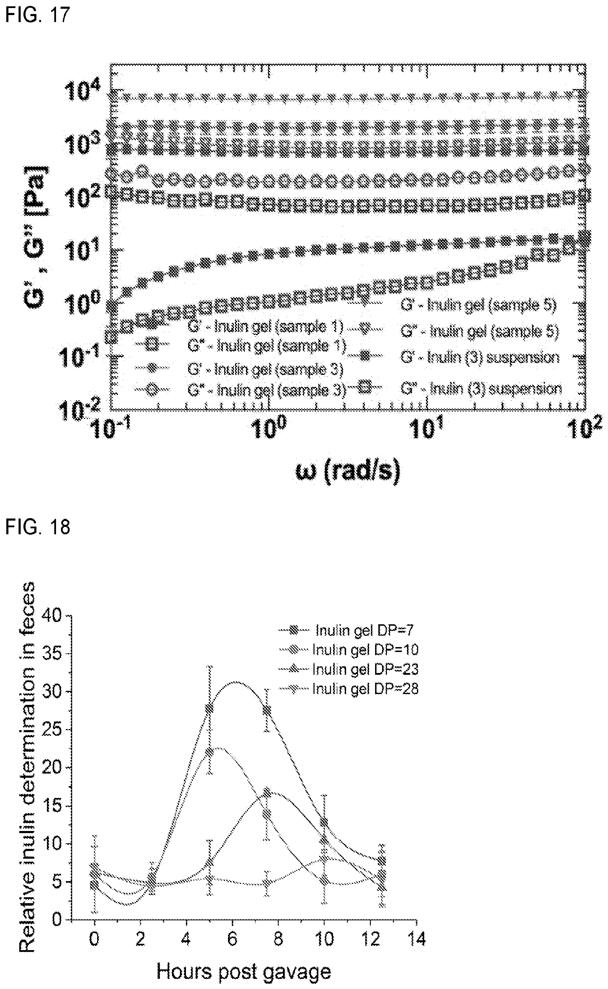

FIG. 17: Gel rheological properties of inulin gel formulations formed with varying average DP were measured by frequency sweeps. G' represents elastic modulus, G" represents viscous modulus. Inulin gel sample #1 had the average DP≈7. Inulin gel sample #3 had the average DP≈23. Inulin gel sample #5 had the average DP≈28.
FIG. 18: Naive mice were orally gavaged with inulin gel with various average DPs, and inulin content in fecal samples were determined over time. Data shows mean ± SEM.

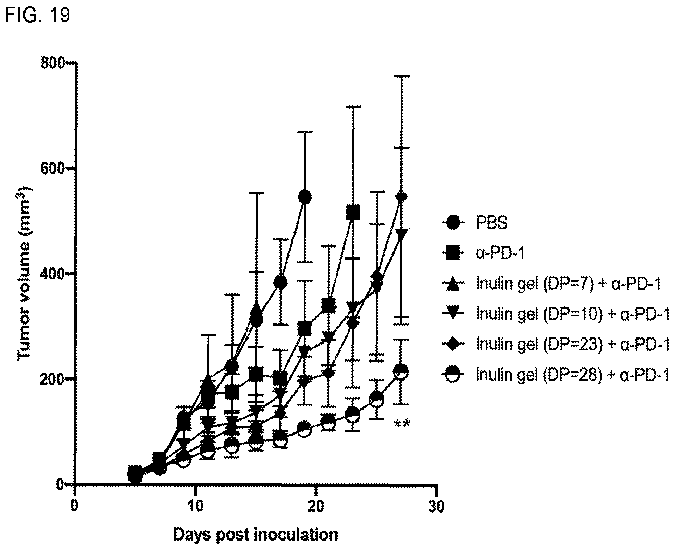

FIG. 19: Balb/c mice bearing s.c. flank CT26 tumors were treated with oral gavage of inulin gels formed with various DPs (60 mg/dose) plus intraperitoneal administration of α-PD-1 (100 ug/dose). Animals were monitored for tumor growth. Data shows mean ± SEM.

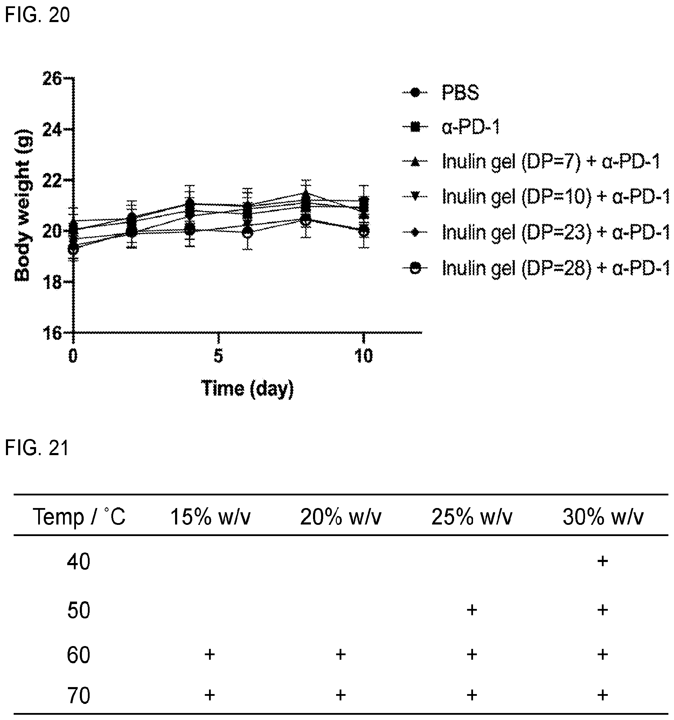

FIG. 20: Balb/c mice bearing s.c. flank CT26 tumors were treated with oral gavage of inulin gels formed with various DPs (60 mg/dose) plus intraperitoneal administration of α-PD-1 (100 ug/dose). Animals were monitored for body weight changes. Data shows mean ± SEM.
FIG. 21: Concentration and temperature dependency of inulin gel formation. Inulin with DP 23 was mixed with water, giving desired concentrations of 15, 20, 25, and 30% w/v. Mixtures were heated to 40, 50, 60, 70 °C respectively for 5 minutes, followed by cooling. Gel formation was examined.

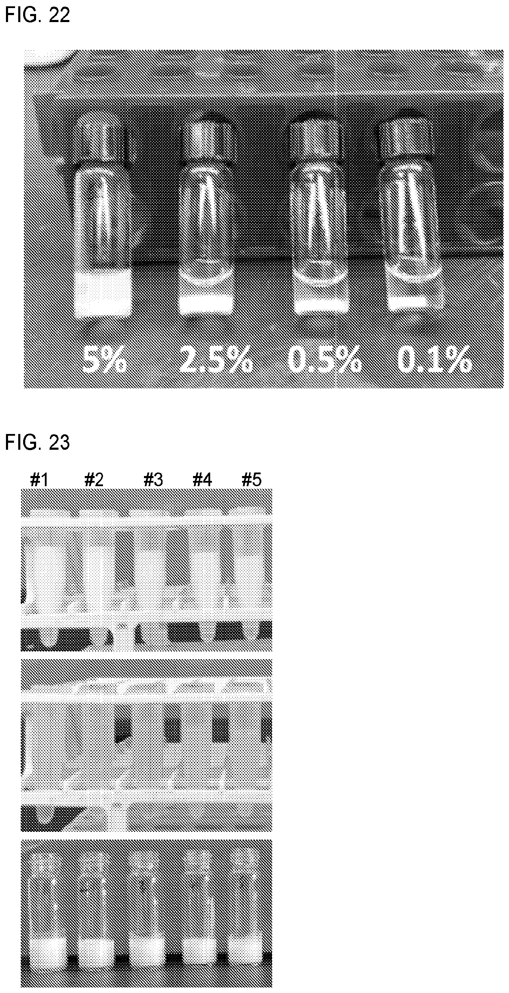

FIG. 22: Food grade potato starch was pre-hydrated at concentrations of 0.1, 0.5, 2.5, and 5% w/v in water, followed by gentle stirring at 70 °C. After cooling, gel formation was examined.
FIG. 23: The gelation process for new fiber gels. #1 inulin gel, #2, inulin gel containing resistant potato starch, #3 inulin gel containing guar gum, #4 inulin gel containing pectin, and #5 inulin gel containing bean gum. The ingredients were first mixed in water (top panel), heated at 70°C for 5 minutes (middle panel), followed by cooling at room temperature overnight, leading to the formation of fiber gels (bottom panel).

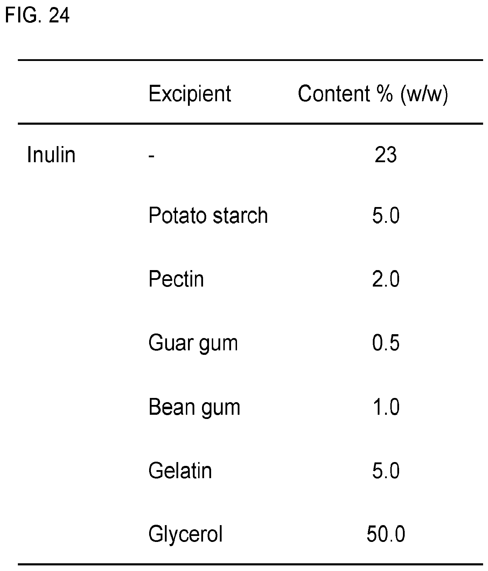

FIG. 24: Inulin gel was formed after adding various amounts of natural fibers or excipients. The values shown are the maximum amount of each ingredient that could be added to 23% w/w inulin gel without disrupting the gel formation.

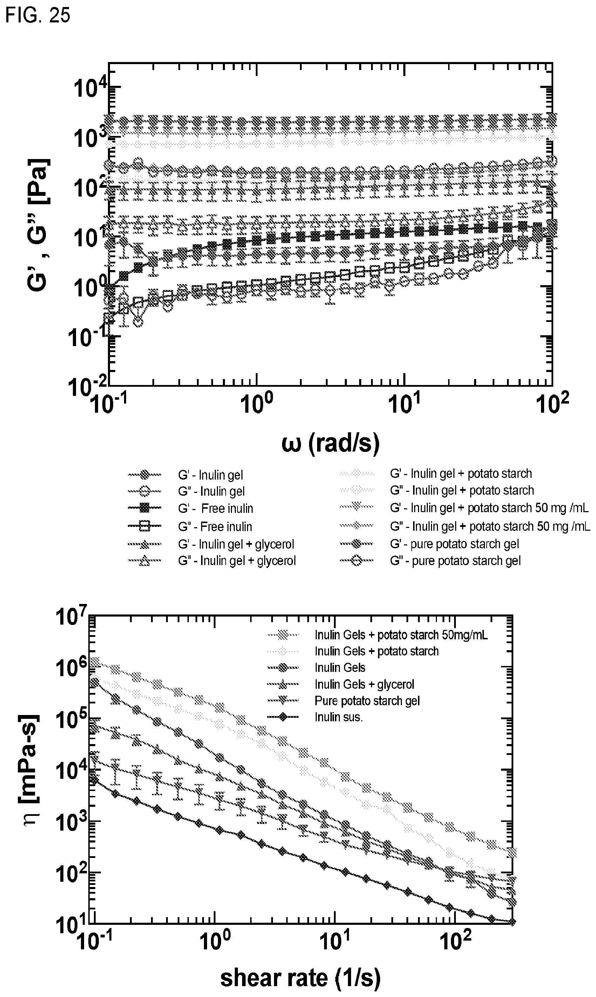

FIG. 25: Gel rheological properties of different fiber gel formulations. Viscoelasticity measured by frequency sweeps (Left) and flow curves for the viscosity (Right).

### DEFINITIONS

For the purposes of promoting an understanding of the principles of the present disclosure, reference will now be made to preferred embodiments and specific language will be used to describe the same.

Articles "a" and "an" are used herein to refer to one or to more than one (i.e. at least one) of the grammatical object of the article. By way of example, "an element" means at least one element and can include more than one element.

"About" is used to provide flexibility to a numerical range endpoint by providing that a given value may be "slightly above" or "slightly below" the endpoint without affecting the desired result.

The use herein of the terms "including," "comprising," or "having," and variations thereof, is meant to encompass the elements listed thereafter and equivalents thereof as well as additional elements. Embodiments recited as "including," "comprising/* or "having" certain elements are also contemplated as "consisting essentially of and "consisting of those certain elements.

Recitation of ranges of values herein are merely intended to serve as a shorthand method of referring individually to each separate value falling within the range, unless otherwise-Indicated herein, and each separate value is incorporated into the specification as if it were individually recited herein. For example, if a concentration range is stated as 1% to 50%, it is intended that values such as 2% to 40%, 10% to 30%, or 1% to 3%, etc., are expressly enumerated in this specification. These are only examples of what is specifically intended, and all possible combinations of numerical values between and including the lowest value and the highest value enumerated are to be considered to be expressly stated in this disclosure.

The transitional term "comprising," which is synonymous with "including," "containing," or "characterized by," is inclusive or open-ended and does not exclude additional, unrecited elements or method steps. By contrast, the transitional phrase "consisting of excludes any element, step, or ingredient not specified in the claim. The transitional phrase "consisting essentially of limits the scope of a claim to the specified materials or steps "and those that do not materially affect the basic and novel characteristic(s)" of the claimed invention.

As used herein, the terms "immune checkpoint inhibitors" (ICIs), "checkpoint inhibitors," and the like refer to compounds that inhibit the activity of control mechanisms of the immune system. Immune system checkpoints, or immune checkpoints, are inhibitory pathways in the immune system that generally act to maintain self-tolerance or modulate the duration and amplitude of physiological immune responses to minimize collateral tissue damage. ICIs can inhibit an immune system checkpoint by inhibiting the activity of a protein in the pathway. ICI proteins include, but are not limited to, CD80, CD28, CD86, cytotoxic T-lymphocyte-associated protein 4 (CTLA-4), PD-L1, PD-L2, PD-1, Ligand of Inducible T-cell costimulator (L-ICOS), Inducible T-cell co-stimulator (ICOS), CD276, and V-set domain containing T cell activation inhibitor 1 (VTCN1). As such, ICI inhibitors include antagonists of, for example, ICIs such as CTLA4, PD1, or PD-L1. For example, antibodies that bind to CTLA4, PD-1, or PD-L1 and antagonize their function are ICI inhibitors. Moreover, any molecule (e.g., peptide, nucleic acid, small molecule, etc.) that inhibits the inhibitory function of an ICI is an ICI inhibitor.

A "subject" can be a vertebrate, a mammal, or a human. Mammals include, but are not limited to, farm animals, sport animals, pets, primates, mice and rats. In one aspect, a subject is a human.

Unless otherwise defined, all technical terms used herein have the same meaning as commonly understood by one of ordinary skill in the art to which this disclosure belongs.

### DETAILED DESCRIPTION

Emerging research suggest that the gut microbiome has a crucial role in modulating immune responses. Indeed, there have been efforts to use fecal microbiomta transfer or probiotics to boost the effiacy of immune checkpoint blockade.

Immune checkpoint therapy, which targets regulatory pathways in T cells to enhance antitumor immune responses, has led to important clinical advances and provides a new weapon against cancer. This therapy has elicited durable clinical responses and, in a fraction of patients, long-term remissions where patients exhibit no clinical signs of cancer for many years.

A number of these immune checkpoints, such as CTLA-4 (cytotoxic T-lymphocyte antigen 4), and PD-1 (programmed death 1) are known to prevent T cells from attacking tumor cells. Therapies comprising antibodies that target CTLA-4 (e.g., ipilimumab) and PD-1 (e.g., nivolumab and pembrolizumab) are known to boost the immune response against cancer cells and have shown efficacy in treating certain cancers. However, the cost and required route of administration (IV), coupled with deleterious side effects, are a hurdle to patient compliance.

Experiments conducted during the course of developing embodiments for the present invention identified new compounds from FDA approved drugs or diet supplements (e.g., prebiotic agents) that can be ingested to improve the efficacy of immune checkpoint therapies. It was shown that oral formulations of an agent (e.g., fiber containing prebiotic agent (e.g., epigallocatechin gallate (EGCG), fucoidan, potato starch, oligofructose and inulin)) (e.g., melatonin) can alter the gut microbiome so that beneficial bacteria increase in frequency and synergize with immune checkpoint blockers given by the systemic injection. Indeed, it was shown that concurrent administration of prebiotic agents (e.g., inulin) with an immunotherapy (e.g., α-PD-1 therapy) resulted in stronger anti-tumor efficacy, stronger immune response (e.g. AH1-specific CD8⁺ T cells frequency among PBMCs), inhibition of tumor growth, enhanced intratumoral infiltration of T cells, higher IFN-γ expression in splenoic CD8⁺ T cells, and increased abundance of Akkermansia, Lactobacillus, Ruminococcus, Roseburia, and Butyricicoccus within the subject's gut microbiome.

Accordingly, the present disclosure relates generally to an agent capable of increasing the efficacy of immunotherapies and vaccines. In particular, the present disclosure relates to elevating the richness and diversity of a subject's gut microbiome through administration of an agent (e.g., fiber containing prebiotic agent (e.g., epigallocatechin gallate (EGCG), fucoidan, potato starch, oligofructose and inulin)) (e.g., melatonin) with administering an immunotherapy or vaccine. The present invention relates to an agent capable of elevating the richness and diversity of the human subject's gut microbiome for use in the treatment of a cancer, the treatment comprising, administering to a human subject suffering from cancer a cancer immunotherapy or vaccine, and the agent capable of elevating the richness and diversity of the human subject's gut microbiome, as defined in the claims.

In certain embodiments, useful for understanding the invention, a method for increasing the efficacy of a cancer immunotherapy is provided through administration of 1) a cancer immunotherapy to a subject, and 2) administration of an agent capable of elevating the richness and diversity of the subject's gut microbiome.

In certain embodiments, useful for understanding the invention, methods of inhibiting the ability of a cancer cell to induce immune dysfunction are provided, comprising administration of 1) a cancer immunotherapy to a subject, and 2) administration of an agent capable of elevating the richness and diversity of the subject's gut microbiome.

In certain embodiments, useful for understanding the invention, a method for increasing the efficacy of a vaccine (e.g., cancer vaccine) (e.g., vaccines against infectious pathogens) is provided through administration of 1) a vaccine to a subject, and 2) administration of an agent capable of elevating the richness and diversity of the subject's gut microbiome.

In certain embodiments, useful for understanding the invention, methods of inhibiting the ability of a cancer cell to induce immune dysfunction are provided, comprising administration of 1) a cancer vaccine to a subject, and 2) administration of an agent capable of elevating the richness and diversity of the subject's gut microbiome.

In certain embodiments, useful for understanding the invention, methods for treating cancer are provided, comprising administration of 1) a cancer immunotherapy to a subject, and 2) administration of an agent capable of elevating the richness and diversity of the subject's gut microbiome.

In certain embodiments, useful for understanding the invention, methods for treating cancer are provided, comprising administration of 1) a cancer vaccine to a subject, and 2) administration of an agent capable of elevating the richness and diversity of the subject's gut microbiome.

In certain embodiments, useful for understanding the invention, methods for treating a condition characterized with dysregulated gut microbiome activity are provided, comprising administration of an agent capable of elevating the richness and diversity of the subject's gut microbiome.

In some embodiments, such administration of the agent capable of elevating the richness and diversity of the subject's gut microbiome occurs prior to, concurrent with, or after administration of the vaccine (e.g., cancer vaccine) (e.g., vaccines against infectious pathogens) or cancer immunotherapy. In some embodiments, such administration of the agent capable of elevating the richness and diversity of the subject's gut microbiome occurs prior to administration of the vaccine (e.g., cancer vaccine) (e.g., vaccines against infectious pathogens) or cancer immunotherapy. In some embodiments, such administration of the agent capable of elevating the richness and diversity of the subject's gut microbiome occurs prior to, concurrent with, and after administration of the vaccine (e.g., cancer vaccine) (e.g., vaccines against infectious pathogens) or cancer immunotherapy.

Such methods are not limited to a particular subject. In some embodiments, the subject is a mammal. In some embodiments, the subject is a human subject. In some embodiments, the subject is a human subject diagnosed with cancer. In some embodiments, the subject is a human subject at risk for developing cancer. In some embodiments, the subject is a human subject having a condition characterized with dysregulated gut microbiome activity.

According to the disclosure, such methods are not limited to a particular agent capable of elevating the richness and diversity of a subject's gut microbiome. In some embodiments of the disclosure, the agent is a fiber containing prebiotic agent (e.g., epigallocatechin gallate (EGCG), fucoidan, potato starch, oligofructose and inulin). In some embodiments, the agent is melatonin. In some embodiments, the agent is capable of inducing increased abundance of Akkermansia, Lactobacillus, Ruminococcus, Roseburia, and Butyricicoccus within the subject's gut microbiome. In some embodiments of the disclosure, the fiber containing prebiotic agent is a gel-based fiber containing prebiotic agent (e.g., gel based inulin). According to the invention, the agent capable of elevating the richness and diversity of a subject's gut microbiome is a fiber containing prebiotic agent,which is a gel-based inulin formulation, having an average degree of polymerization at or higher than 20 and at or less than 47.

According to the disclosure, among the prebiotics suitable for treatment, as either part of any food or as supplement, are included the following components: 1,4-dihydroxy-2-naphthoic acid (DHNA), Inulin, trans-Galactooligosaccharides (GOS), Lactulose, Mannan oligosaccharides (MOS), Fructooligosaccharides (FOS), Neoagaro-oligosaccharides (NAOS), Pyrodextrins, Xylo-oligosaccharides (XOS), Isomalto-oligosaccharides (IMOS), Amylose-resistant starch, Soybean oligosaccharide (SBOS), Lactitol, Lactosucrose (LS), Isomaltulose (including Palatinose), Arabinoxylooligosaccharides (AXOS), Raffinose oligosaccharides (RFO), Arabinoxylans (AX), Polyphenols or any another compound capable of changing the microbiota composition with a desirable effect.

According to the disclosure, such methods are not limited to a specific cancer immunotherapy.

In some embodiments, the cancer immunotherapy is one or more immune checkpoint inhibitor (ICI) inhibitors.

ICIs include any agent that blocks or inhibits in a statistically significant manner, the inhibitory pathways of the immune system. Illustrative ICIs that may be targeted for blocking or inhibition include, but are not limited to, CTLA-4, PDL1, PDL2, PD1, B7-H3, B7-H4, BTLA, HVEM, GAL9, LAG3, TIM3, VISTA, KIR, 2B4 (belongs to the CD2 family of molecules and is expressed on all NK, γδ, and memory CD8⁺ (αβ) T cells), CD160 (also referred to as BY55), CGEN-15049, CHK 1 and CHK2 kinases, A2aR and various B-7 family ligands. B7 family ligands include, but are not limited to, B7-1, B7-2, B7-DC, B7-H1, B7-H2, B7-H3, B7-H4, B7-H5, B7-H6 and B7-H7. ICIs include antibodies, or antigen binding fragments thereof, other binding proteins, biologic therapeutics or small molecules, that bind to and block or inhibit the activity of one or more of CTLA-4, PDL1, PDL2, PD1, BTLA, HVEM, TIM3, GAL9, LAG3, VISTA, KIR, 2B4, CD160 and CGEN-15049. Illustrative ICIs include Tremelimumab (CTLA-4 blocking antibody), anti-OX40, PD-L1 monoclonal Antibody (Anti-B7-H1; MEDI4736), MK-3475 (PD-1 blocker), Nivolumab (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and Yervoy/ipilimumab (anti-CTLA-4 checkpoint inhibitor). Checkpoint protein ligands include, but are not limited to PD-L1, PD-L2, B7-H3, B7-H4, CD28, CD86 and TIM-3.

In some embodiments, the present invention covers the use of a specific class of ICIs are drugs that block the interaction between immune checkpoint receptor programmed cell death protein 1 (PD-1) and its ligand PD-L1 (see, Mullard, Nature Reviews: Drug Discovery (2013)), 12:489-492. PD-1 is expressed on and regulates the activity of T-cells. Specifically, when PD-1 is unbound to PDL-1, the T-cells can engage and kill target cells. However, when PD-1 is bound to PDL-1 it causes the T-cells to cease engaging and killing target cells. Furthermore, unlike other checkpoints, PD-1 acts proximately such the PDLs are overexpressed directly on cancer cells which leads to increased binding to the PD-1 expressing T-cells.

In some embodiments, ICIs which are antibodies are provided that can act as agonists of PD-1, thereby modulating immune responses regulated by PD-1. In one embodiment, the anti-PD-1 antibodies can be antigen-binding fragments. Anti-PD-1 antibodies disclosed herein are able to bind to human PD-1 and agonize the activity of PD-1, thereby inhibiting the function of immune cells expressing PD-1.

In some embodiments, the present invention covers the use of a specific class of ICIs that are drugs that inhibit CTLA-4. Suitable anti-CTLA4 antagonist agents for use in the invention, include, without limitation, anti-CTLA4 antibodies, human anti-CTLA4 antibodies, mouse anti-CTLA4 antibodies, mammalian anti-CTLA4 antibodies, humanized anti-CTLA4 antibodies, monoclonal anti-CTLA4 antibodies, polyclonal anti-CTLA4 antibodies, chimeric anti-CTLA4 antibodies, MDX-010 (ipilimumab), tremelimumab, anti-CD28 antibodies, anti-CTLA4 adnectins, anti-CTLA4 domain antibodies, single chain anti-CTLA4 fragments, heavy chain anti-CTLA4 fragments, light chain anti-CTLA4 fragments, inhibitors of CTLA4 that agonize the co-stimulatory pathway, the antibodies disclosed in PCT Publication No. WO 2001/014424, the antibodies disclosed in PCT Publication No. WO 2004/035607, the antibodies disclosed in U.S. Publication No. 2005/0201994, and the antibodies disclosed in granted European Patent No. EP 1212422 B1. Additional CTLA-4 antibodies are described in U.S. Pat. Nos. 5,811,097, 5,855,887, 6,051,227, and 6,984,720; in PCT Publication Nos. WO 01/14424 and WO 00/37504; and in U.S. Publication Nos. 2002/0039581 and 2002/086014. Other anti-CTLA-4 antibodies that can be used in the present invention include, for example, those disclosed in: WO 98/42752; U.S. Pat. Nos. 6,682,736 and 6,207,156; Hurwitz et al., Proc. Natl. Acad. Sci. USA, 95(17):10067-10071 (1998); Camacho et al., J. Clin. Oncology, 22(145):Abstract No. 2505 (2004) (antibody CP-675206); Mokyr et al., Cancer Res., 58:5301-5304 (1998), and U.S. Pat. Nos. 5,977,318, 6,682,736, 7,109,003, and 7,132,281.

Additional anti-CTLA4 antagonists include, but are not limited to, the following: any inhibitor that is capable of disrupting the ability of CD28 antigen to bind to its cognate ligand, to inhibit the ability of CTLA4 to bind to its cognate ligand, to augment T cell responses via the co-stimulatory pathway, to disrupt the ability of B7 to bind to CD28 and/or CTLA4, to disrupt the ability of B7 to activate the co-stimulatory pathway, to disrupt the ability of CD80 to bind to CD28 and/or CTLA4, to disrupt the ability of CD80 to activate the co-stimulatory pathway, to disrupt the ability of CD86 to bind to CD28 and/or CTLA4, to disrupt the ability of CD86 to activate the co-stimulatory pathway, and to disrupt the co-stimulatory pathway, in general from being activated. This necessarily includes small molecule inhibitors of CD28, CD80, CD86, CTLA4, among other members of the co-stimulatory pathway; antibodies directed to CD28, CD80, CD86, CTLA4, among other members of the co-stimulatory pathway; antisense molecules directed against CD28, CD80, CD86, CTLA4, among other members of the co-stimulatory pathway; adnectins directed against CD28, CD80, CD86, CTLA4, among other members of the co-stimulatory pathway, RNAi inhibitors (both single and double stranded) of CD28, CD80, CD86, CTLA4, among other members of the co-stimulatory pathway, among other anti-CTLA4 antagonists.

In one embodiment, the present invention covers the use of a specific class of ICI are drugs that inhibit TIM-3. Blocking the activation of TIM-3 by a ligand, results in an increase in Th1 cell activation. Furthermore, TIM-3 has been identified as an important inhibitory receptor expressed by exhausted CD8+ T cells. TIM-3 has also been reported as a key regulator of nucleic acid mediated antitumor immunity. In one example, TIM-3 has been shown to be upregulated on tumor-associated dendritic cells (TADCs).

Such methods are not limited to the use of a particular cancer vaccine. Indeed, one approach that has been pursued for cancer immunotherapy is the area covered by the term "tumor vaccines" or "cancer vaccines" which includes immunization with tumor specific or overexpressed antigens. In this approach, an antigen or antigens specific for, or overexpressed in, tumor cells are injected alone, with adjuvants, as part of a microorganism that delivers the antigen (for example, Listeria Monocytogenes), or after incubation ex-vivo with immune cells (including but not limited to dendritic cells) in order to elicit cellular and/or humoral immune responses.

In some embodiments, the cancer is carcinoma. Carcinomas are cancers of epithelial origin. In some embodiments, the carcinoma is selected from the group consisting of acinar carcinoma, acinous carcinoma, alveolar adenocarcinoma, carcinoma adenomatosum, adenocarcinoma, carcinoma of adrenal cortex, alveolar carcinoma, alveolar cell carcinoma, basal cell carcinoma, carcinoma basocellular, basaloid carcinoma, basosquamous cell carcinoma, breast carcinoma, bronchioalveolar carcinoma, bronchiolar carcinoma, cerebriform carcinoma, cholangiocellular carcinoma, chorionic carcinoma, colloid carcinoma, comedocarcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epibulbar carcinoma, epidermoid carcinoma, carcinoma epitheliate adenoids, carcinoma exulcere, carcinoma fibrosum, gelatinform carcinoma, gelatinous carcinoma, giant cell carcinoma, gigantocellulare, glandular carcinoma, granulose cell carcinoma, hair matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, lentivular carcinoma, carcinoma lenticulare, lipomatous carcinoma, lymphoepithelial carcinoma, carcinoma mastotoids, carcinoma medullare, medullary carcinoma, carcinoma melanodes, melanotonic carcinoma, mucinous carcinoma, carcinoma muciparum, carcinoma mucocullare, mucoepidermoid carcinoma, mucous carcinoma, carcinoma myxomatodes, masopharyngeal carcinoma, carcinoma nigrum, oat cell carcinoma, carcinoma ossificans, osteroid carcinoma, ovarian carcinoma, papillary carcinoma, periportal carcinoma, preinvasive carcinoma, prostate carcinoma, renal cell carcinoma of kidney, reserve cell carcinoma, carcinoma sarcomatodes, scheinderian carcinoma, scirrhous carcinoma, carcinoma scrota, signet-ring cell carcinoma, carcinoma simplex, small cell carcinoma, solandoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberrosum, tuberous carcinoma, verrucous carcinoma, carcinoma vilosum.

In some embodiments, the cancer is a sarcoma. Sarcomas are mesenchymal neoplasms that arise in bone and soft tissues. In some embodiments, the sarcoma is selected from liposarcomas (including myxoid liposarcomas and pleomorphic liposarcomas), leiomyosarcomas, rhabdomyosarcomas, neurofibrosarcomas, malignant peripheral nerve sheath tumors, Ewing's tumors (including Ewing's sarcoma of bone, extraskeletal or non-bone) and primitive neuroectodermal tumors (PNET), synovial sarcoma, hemangioendothelioma, fibrosarcoma, desmoids tumors, dermatofibrosarcoma protuberance (DFSP), malignant fibrous histiocytoma (MFH), hemangiopericytoma, malignant mesenchymoma, alveolar soft-part sarcoma, epithelioid sarcoma, clear cell sarcoma, desmoplastic small cell tumor, gastrointestinal stromal tumor (GIST) and osteosarcoma (also known as osteogenic sarcoma-skeletal and extraskeletal, and chondrosarcoma.

In some embodiments, the cancer is a refractory or a responding cancer. As used herein, a refractory cancer is a cancer that is resistant to the ordinary standards of care prescribed. These cancers, although initially responsive to treatment, recur and/or may be completely non responsive to the treatment.

**In** some embodiments, the cancer is an immunogenic cancer. Examples of immunogenic cancers include malignant melanoma and renal cell carcinoma, Mantel cell lymphoma, follicular lymphoma, diffuse large B-cell lymphoma, T-cell acute lymphoblastic leukemia, Burkitt Lymphoma, myeloma, immunocytoma, acute promyelocyte leukemia, chronic myeloid/acute lymphoblastic leukemia, acute leukemia, B-cell acute lymphoblastic leukemia, anaplastic large cell leukemia, myelodysplasia syndrome/acute myeloid leukemia, non-Hodgkin's lymphoma, chronic lymphocytic leukemia, acute myelogenous leukemia (AML), common (pre-B) acute lymphocytic leukemia, malignant melanoma, T-cell lymphoma, leukemia, B-cell lymphoma, epithelial malignancies, lymphoid malignancies, gynecologic carcinoma, biliary adenocarcinomas and ductal adenocarcinomas of the pancreas.

In some embodiments, such methods further comprise administering other therapies such as, for example, radiation therapy, surgery, conventional chemotherapy or with a combination of one or more additional therapies. Such other active ingredient includes, but is not limited to glutathione antagonists, angiogenesis inhibitors, chemotherapeutic agent(s) and antibodies (e.g., cancer antibodies). The agents described in this invention may be administered simultaneously or sequentially. The separation in time between administrations may be minutes, hours, days or it may be longer.

For example, the agent capable of elevating the richness and diversity of the subject's gut microbiome and the cancer immunotherapy or cancer vaccine can be administered before, after, or simultaneously with an additional chemotherapeutic and/or cytotoxic agents such as alkylating agents (e.g., chlorambucil, cyclophosphamide, ccnu, melphalan, procarbazine, thiotepa, bcnu, and busulfan), antimetabolites (e.g., 6-mercaptopurine and 5-fluorouracil), anthracyclines (e.g., daunorubicin, doxorubicin, idarubicin, epirubicin, and mitoxantrone), antitumor antibiotics (e.g., bleomycin), monoclonal antibodies (e.g., alemtuzumab, bevacizumab, cetuximab, gemtuzumab, ibritumomab, panitumumab, rituximab, tositumomab, and trastuzumab), platinums (e.g., cisplatin, oxaliplatin, and carboplatin), plant alkaloids (e.g., vincristine), topoisomerase I or II inhibitors (e.g., irinotecan, topotecan, amsacrine, etoposide, etoposide phosphate, and teniposide), vinca alkaloids (e.g., vincristine, vinblastine, vinorelbine, and vindesine), taxanes (e.g., paclitaxel and docetaxel), epipodophyllotoxins (e.g., etoposide and teniposide), nucleoside analogs, and angiogenesis inhibitors (e.g., Avastin (beracizumab), a humanized monoclonal antibody specific for VEGF-A).

Examples of glutathione antagonists include but are not limited to buthionine sulfoximine, cyclophosphamide, ifosphamide, actinomycin-d and N-(4-hydroxyphenyl) retinamide (4-HPR). Examples of angiogenesis inhibitors include but are not limited to 2-methoxyestradiol(2-ME), AG3340, Angiostatin, antithrombin-III, Anti-VEGF antibody, Batimastat, bevacizumab (Avastin), BMS-275291, CA1, Canstatin, combretastatin, Combretastatin-A4 phosphate, CC-5013, captopril, celecoxib, Dalteparin, EMD121974, Endostatin, Erlotinib, Gefitinib, Genistein, Halofuginone, ID 1, ID3, IM862, Imatinib mesylate, Inducible protein-10, Interferon-alpha, Interleukin-12, Lavendustin-a, LY317615, or AE-941, Marimastat, Mapsin, Medroxyprogesterone acetate, Meth-1, Meth-2, Neovastat, Osteopontin cleaved product, PEX, Pigment epithelium growth factor (PEGF), platelet growth factor 4, prolactin fragment, proliferin-related protein (PRP), PTK787/ZK222584, recombinant human platelet factor-4(rPF4), restin, squalamine, SU5416, SU6668, Suramin, Taxol, Tecogalan, Thalidomide, Tetrathiomolybdate (TM), Thrombospondin, TNP-470, Troponin I, Vasostatin, VEGF1, VEGF-TPvAP and ZD6474. In some embodiment the angiogenesis inhibitor is a VRGF antagonist. The VEGF antagonist may be a VEGF binding molecule. VEGF binding molecule include VEGF antibodies, or antigen binding fragment (s) thereof. One example of a VEGF antagonist is NeXstar.

Examples of categories of chemotherapeutic agents that may be used in any of the methods or agents disclosed herein include, but are not limited to, DNA damaging agents and these include topoisomerase inhibitors (e.g., etoposide, camptothecin, topotecan, irinotecan, teniposide, mitoxantrone), anti-microtubule agents (e.g., vincristine, vinblastine), antimetabolite agents (e.g., cytarabine, methotrexate, hydroxyurea, 5-fluorouracil, flouridine, 6-thioguanine, 6-mercaptompurine, fludarabine, pentostatin, chlorodeoxyadenosine), DNA alkylating agents (e.g., cisplatin, mecholorethamine, cyclophosphamide, ifosphamide, melphalan, chlorambucil, busulfan, thiotepa, carmustine, lomustine, carboplatin, dacarbazine, procarbazine) and DNA strand break inducing agents (e.g., bleomycin, doxorubicin, daunorubicin, idarubicin, mitomycin C).

Chemotherapeutic agents include synthetic, semisynthetic and naturally derived agents. Important chemotherapeutic agents include, but are not limited to, Avicine, Aclarubicin, Acodazole, Acronine, Adozelesin, Adriamycin, aldesleukin, Alitretinoin, AUopurinol sodium, Altretamine, Ambomycin, Ametantrone acetate, Aminoglutethimide, Amsacrine, Anastrazole, Annonaceous Acetogenins, Anthramycin, Asimicin, Asparaginase, asperlin, Azacitidine, azetepa, Azotomycin, batimastat, benzodepa, bexarotene, Bicalutamide, Bisantrene, Bisnafide, Bizelesin, Bleomycin, Brequinar, Bropirimine, Bullatacin, Busulfan, Cabergoline, cactinomycin, calusterone, caracemide, carbetimer, carboplatin, carmustine, carubicin, carzelesin, cedefingol, chlorambucil, celecoxib, cirolemycin, cisplatin, cladribine, crisnatol, cyclophosphamide, cytarabine, dacarbazine, DACA, dactinomycin, Daunorubicin, daunomycin, Decitabine, denileukin, Dexormaplatin, Dezaguanine, Diaziquone, Docetaxel, Doxorubicin, Droloxifene, Dromostalone, Duazomycin, Edatrexate, Eflornithine, Elsamitrucin, Estramustine, Etanidazole, Etoposide, Etoprine, Fadrozole, Fazarabine, Fenretinide, Floxuridine, Fludarabine, Fluorouracil, Flurocitabine, 5-FdUMP, Fosquidone, Fosteuecine, FK-317, FK-973, FR-66979, FR-900482, Gemcitabine, Gemtuzumab, Ozogamicin, Gold Aul 98, Goserelin, Guanacone, Hydroxyurea, Idarubicin, Ilmofosine, Interferon alpha and analogs, Iproplatin, irinotecan, Lanreotide, Letrozole, Leuprolide, Liarozole, Lometrexol, Lomustine, Losoxantrone, masoprocol, Maytansine, Mechlorethamine, Megestrol, Melengestrol, Melphalan, Menogaril, Metoprine, maturedepa, mitindomide, Mitocarcin, Mitogillin, Mitomalacin, Mitomycin, Mitomycin C, Mitosper, Mitotane, Mitoxantrone, Mycophenolic acid, Nocodazole, Nogalamycin, Oprelvekin, ormaplatin, Oxisuran, Paclitaxel, pamidronate, pegaspargase, Peliomycin, Pentamustine, Peplomycin, Perfosfamide, Pipobroman, Piposulfan, Piroxantrone, Plicamycin, Plomestane, Porfimer, Porfiromycin, Prednimustine, procarbazine, Puromycin, Pyrazofurin, Riboprine, Rituximab, Rogletimide, Rolliniastatin, safingol, Samarium, Semustine, Simtrazene, Sparfosate, Sparsomycin, spirogermanium, Spiromustine, Spiroplatin, Squamocin, Squamotacin, streptonigrin, streptozocin, SrC12, Sulphofenur, Talisomycin, Taxane, Toxoid, Tecoglan, Tegafur, teloxantrone, Temoporfin, teniposide, Teroxirone, Testolactone, Thiamiprine, Thiotepa, Thymitaq, Tiazofurin, Tirapazamine, Tomudex, Top-53, Topotecan, Toremixifme, Trastuzumab, Trestolone, triciribine, Triciribine, Trimetrexate, trimetrexate glucuronate, Triptorelin, Tubulozole, uracil mustard, Uredepa, valrubicin, vapreotide, Vinblastine, Vincristine, Vindesine, Vinepidine, Vinglycinate, Vinleurosine, Vinorelbine, Vinrosidine, Vinzolidine, Vorozole, Zeniplatin, Zinostatin, Zorubicin, 2-cholrodeoxyrubicine, 2'-deoxyformycin, 9-aminocamptothecin, raltitrexed, N-propargyl-5,8-didezafolic acid, 2-cholo-2'arabinofluoro-2' deoxyadenosine, 2-cholo-2'-deoxyadenosine, anisomycin, Trichostatin, hPRL-G129R, CEP-751, Linomide, Sulfur mustard, nitrogen mustard, N-methyl-N-nitrosourea, fotemustine, Streptozotocin, dacarbazine, mitozolomide, temozolomide, AZQ, ormaplatin, CI-973, DWA21 14R, JM216, JM335, Bisplatinum, Tomudex, azacitidine, cytrabincine, gemcitabine, 6-mercaptopurine, Hypoxanthine, Teniposide, CPT-11, Doxorubicin, Daunorubicin, Epirubicin, darubicin, losoxantrone, amsacrine, pyrazoloacridine, all trans retinol, 14-hydroxy-retro-retinol, all-trans retinoic acid, N-(4-hydroxyphenyl) retinamide, 13-cisretinoic acid, 3-methyl TTNEB, 9-cisretenoic acid, fludarabine, and 2-Cda.

Other chemotherapeutic agent include: 20-epi1,25-dihydroxyvitamin-D3, 5-ethynyl uracil, abiraterone, aclarubicin, acylfulvene, adecylpenol, adozelesin, aldesleukin, ALL-TK antagonists, altretamine, ambumastine, amidox, amifostine, amino levulinic acid, anagrelide, anastrozole, andrographolide, angiogenesis inhibitors, antagonist D, antagonists D, antarelix, anti-dorsalizing morphogenetic protein-1, antiandrogen, antiestrogen, antineoplastone, antisense oligonucleotides, aphidicolin, apoptosis gene modulators, apoptosis regulators, apurinic acid, ara-cdp-dl-PTBA, arginine aminase, asulacrine, atamestine, atrimustine, axinamastine 1 and axinamastine 2, axinamastine 3, azasetron, azatoxin, azatyrosine, baccatin III derivatives, balanol, BCR/ABL antagonist, benzochlorins, benzoylsaurosporine, beta lactam derivatives, beta-alethine, perillyl alcohol, phenozenomyein, phenyl acetate, phosphatase inhibitors, picibanil, pilocarbine and salts or analogs thereof, pirarubucin, piritrexim, placetin A, placetin B, plasminogen activator inhibitor, platinum complex, phenyl ethyl isothiocyanate and analogs thereof, platinum compounds, platinum triamine complex, podophylotoxin, porfimer sodium, porphyromycin, propyl bis acridones, prostaglnadins J2, protease inhibitors, protein A based immune modulators, PKC inhibitors, microalgal, protein tyrosine phosphatase inhibitors, purine nucleoside phosphorylase inhibitors, purpurins, pyrazoloacridines, pyridoxylated hemoglobin polyoxyethylene conjugate, raf antagonists, raltitrexed, ramosetron, ras farnesyl protein tranferase inhibitors, rasinhibitors, ras-GAP inhibitors, ratellitptine demethylated, Rhenium Re 186 etidronate, rhizoxine, ribozyme, RII retinide, rogletimide, rosagliatazone and analogs and derivatives thereof, rohitukine, romurtide, roquinimex, rubiginone B1, ruboxyl, safingol, saintopin, SarCNU, sarcophytol A, sargrmostim, sdi 1 mimetics, semustine, senescence derived inhibitor 1, sense oligonucleotide, signal transduction inhibitors, signal transduction modulators, single chain antigen binding protein, sizofiran, sobuzoxane, sodium borocaptate, sodium phenyl acetate, solverol, somatomedin binding protein, sonermin, sparfosic acid, spicamycin D, spiromustin, splenopentine, spongistatin 1, squalamine, stem cell inhibitor, stem cell division inhibitor, stipiamide, stromelysin, sulfinosine, superactive vasoactive intestinal peptide antagonists, suradista, siramin, swainsonine, synthetic glycosaminoglycans, tallimustine, tamoxifen methiodide, tauromustine, tazarotene, tacogalan sodium, tegafur, tellurapyrilium, telomerase inhibitors, temoporfin, tmeozolomide, teniposide, tetrachlorodecaoxide, tetrazomine, thaliblastine, thalidomide, thiocoraline, thrombopoetin and mimetics thereof, thymalfasin, thymopoetin receptor agonist, thymotrinan, thyroid stimulating harmone, tin ethyl etiopurpin, tirapazamine, titanocene and salts thereof, topotecan, topsentin, toremifene, totipotent stem cell factors, translation inhibitors, tretinoin, triacetyluridine, tricribine, trimetrexate, triptorelin, tropisetron, turosteride, tyrosine kinase inhibitors, tyrphostins, UBC inhibitors, ubenimex, urogenital sinus derived growth inhibitory factor, urokinase receptor antagonists, vapreotide, variolin B, vector system, erythrocyte gene therapy, velaresol, veramine, verdins, verteporfin, vinorelbine, vinxaltine, vitaxin, vorozol, zanoterone, zeniplatin, zilascorb and zinostatin.

Other chemotherapeutic agents include: antiproliferative agents (e.g., piritrexim isothiocyanate), antiprostatic hypertrophy agents (sitogluside), Benign prostatic hyperplasia therapy agents (e.g., tomsulosine, RBX2258), prostate growth inhibitory agents (pentomone) and radioactive agents: Fibrinogen 1125, fludeoxyglucose F18, Flurodopa F18, Insulin 1125, Iobenguane 1123, Iodipamide sodium 1131, Iodoantipyrine 1131, Iodocholesterol 1131, Iodopyracet 1125, Iofetamine HCL 1123, Iomethin 1131, Iomethin 1131, Iothalamate sodium 1125, Iothalamate 1131, Iotyrosine 1131, Liothyronine 1125, Merosproprol Hgl 97, Methyl ioodobenzo guanine (MIBG-I131 or MIBGI 123) selenomethionine Se75, Technetium Tc99m furifosmin, technetium Tc99m gluceptate, Tc99m Biscisate, Tc99m disofenin, TC99m gluceptate, Tc99m lidofenin, Tc99m mebrofenin, Tc99m medronate and sodium salts thereof, Tc99m mertiatide, Tc99m oxidronate, Tc99m pentetate and salts thereof, Tc99m sestambi, Tc99m siboroxime, Tc99m succimer, Tc99m sulfur colloid, Tc 99m teboroxime, Tc 99m Tetrofosmin, Tc99m Tiatide, Thyroxine 1125, Thyroxine 1131, Tolpovidone 1131, Triolein 1125 and Treoline 1125, and Treoline 131, MIBG-I123 and MIBG 1131 are especially preferred chemotherapeutic agents for co-administration with the nitrofuran containing pharmaceutical composition of invention.

Another category of chemotherapeutic agents are anticancer supplementary potentiating agents, e.g., antidepressant drugs (Imipramine, desipramine, amitriptyline, clomipramine, trimipramine, doxepin, nortriptyline, protriptyline, amoxapine, and maprotiline), or no-trycyclic anti-depressant drugs (sertraline, trazodone and citalopram), Ca++ antagonists (verapamil, nifedipine, nitrendipine and caroverine), calmodulin inhibitors (prenylamine, trifluoperazine and clomipramine), Amphotericin B, Triparanol analogs (e.g., Tamoxifen), antiarrhythmic drugs (e.g., quinidine), antihypertensive drugs (e.g., reserpine), thiol depleters (e.g., buthionine and sulfoximine) and multiple drug resistance reducing agents such as Cremophor EL.

In some embodiments of the disclosure the condition characterized with dysregulated gut microbiome activity is an autoimmune disease, a neurological disorder, diabetes, and/or obesity. Examples of such conditions include, but are not limited to, rheumatoid arthritis, multiple sclerosis diabetes (e.g., type 1 diabetes mellitus), autoimmune diseases of the thyroid (e.g., Hashimoto's thyroiditis, Graves' disease), thyroid-associated ophthalmopathy and dermopathy, hypoparathyroidism, Addison's disease, premature ovarian failure, autoimmune hypophysitis, pituitary autoimmune disease, immunogastritis, pernicious angemis, celiac disease, vitiligo, myasthenia gravis, pemphigus vulgaris and variants, bullous pemphigoid, dermatitis herpetiformis Duhring, epidermolysis bullosa acquisita, systemic sclerosis, mixed connective tissue disease, Sjogren's syndrome, systemic lupus erythematosus, Goodpasture's syndrome, rheumatic heart disease, autoimmune polyglandular syndrome type 1, Aicardi-Goutières syndrome, Acute pancreatitis Age-dependent macular degeneration, Alcoholic liver disease, Liver fibrosis, Metastasis, Myocardial infarction, Nonalcoholic steatohepatitis (NASH), Parkinson's disease, Polyarthritis/fetal and neonatal anemia, Sepsis, and inflammatory bowel disease.

In some embodiments of the disclosure such methods for treating or preventing conditions characterized with dysregulated gut microbiome activity disorders further comprise administering (e.g., simultaneously or at different times) additional therapeutic agents. Examples of such therapeutic agents include, but are not limited to, disease-modifying antirheumatic drugs (e.g., leflunomide, methotrexate, sulfasalazine, hydroxychloroquine), biologic agents (e.g., rituximab, infliximab, etanercept, adalimumab, golimumab), nonsteroidal anti-inflammatory drugs (e.g., ibuprofen, celecoxib, ketoprofen, naproxen, piroxicam, diclofenac), analgesics (e.g., acetaminophen, tramadol), immunomodulators (e.g., anakinra, abatacept), glucocorticoids (e.g., prednisone, methylprednisone), TNF-α inhibitors (e.g., adalimumab, certolizumab pegol, etanercept, golimumab, infliximab), IL-1 inhibitors, and metalloprotease inhibitors. In some embodiments, the therapeutic agents include, but are not limited to, infliximab, adalimumab, etanercept, parenteral gold or oral gold.

The present invention provides a composition comprising a gel-based inulin formulation having an average degree of polymerization at or higher than 20 and at or less than 47. In some embodiments, the gel-based inulin formulation has an average degree of polymerization at approximately 28 (e.g., 23, 24, 25, 26, 27, 28, 29, 30, 31, 32, 33). In some embodiments, the gel-based inulin formluation comprises one or more prebiotic compounds selected from a fructo-oligosaccharide, a short-chain fructo-oligosaccharide, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber.

In certain embodiments, useful for understanding the invention, a method for increasing the efficacy of a vaccine is provided through administration of 1) a vaccine to a subject, and 2) administration of an agent capable of elevating the richness and diversity of the subject's gut microbiome.

In some embodiments, administration of the agent capable of elevating the richness and diversity of the subject's gut microbiome occurs prior to, concurrent with, and/or after administration of the vaccine.

In some embodiments, administration of the agent capable of elevating the richness and diversity of the subject's gut microbiome occurs concurrent with administration of the vaccine; or administration of the agent capable of elevating the richness and diversity of the subject's gut microbiome occurs prior to administration of the vaccine. In some embodiments, wherein administration of the agent capable of elevating the richness and diversity of the subject's gut microbiome occurs prior to and concurrent with administration of the vaccine.

In some embodiments of the disclosure, the vaccine is a vaccine for treating cancer, and/or a vaccine for treating and/or protecting from infectious pathogens. According to the invention, the vaccine is a vaccine for treating cancer.

In some embodiments, the subject is a human subject.

In some embodiments of the disclosure, the agent capable of elevating the richness and diversity of a subject's gut microbiome is a fiber containing prebiotic agent. In some embodiments, the fiber containing prebiotic agent is selected from epigallocatechin gallate (EGCG), fucoidan, potato starch, oligofructose and inulin. In some embodiments, the fiber containing prebiotic agent is a gel-based inulin formulation. In some embodiments, the gel-based inulin formulation has an average degree of polymerization at or higher than 20 and at or less than 47. In some embodiments, the gel-based inulin formulation has an average degree of polymerization at approximately 28 (e.g., 23, 24, 25, 26, 27, 28, 29, 30, 31, 32, 33). In some embodiments, the gel-based inulin formluation comprises one or more prebiotic compounds selected from a fructo-oligosaccharide, a short-chain fructo-oligosaccharide, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber. In some embodiments, the agent capable of elevating the richness and diversity of a subject's gut microbiome is melatonin.

According to the invention, the agent capable of elevating the richness and diversity of the subject's gut microbiome is administered orally.

In some embodiments, administration of the agent capable of elevating the richness and diversity of the subject's gut microbiome results in increased relative abundance of Akkermansia, Lactobacillus, Ruminococcus, Roseburia, and Butyricicoccus within the gut microbiome of the subject.

In certain embodiments of the disclosure, the present invention provides compositions comprising a prebiotic formulation associated with (e.g., complexed, conjugated, encapsulated, absorbed, adsorbed, admixed) bacteria.

In some embodiments of the disclosure, the prebiotic formulation comprises one or more prebiotic compounds selected from a fructo-oligosaccharide, a short-chain fructo-oligosaccharide, inulin, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber.

In some embodiments, the prebiotic compound is gel-based. In some embodiments, the prebiotic compound is gel-based inulin.

In some embodiments of the disclosure, the bacteria is E. coli. In some embodiments, the bacteria is able to alter the gut microbiome of a subject upon administration to the subject.

In some embodiments, the composition is configured for oral administration to a subject.

In certain embodiments, useful for understanding the invention, methods for colonic delivery of bacteria to a subject are provided, comprising administering to a subject such a comprising a prebiotic formulation associated with (e.g., complexed, conjugated, encapsulated, absorbed, adsorbed, admixed) bacteria (e.g., gel-based inulin).

In certain embodiments, the present disclosure provides compositions comprising a prebiotic formulation associated with (e.g., complexed, conjugated, encapsulated, absorbed, adsorbed, admixed) one or more probiotic cells. In certain embodiments, useful for understanding the invention, methods for increasing the growth of probiotic organisms in the digestive system of a subject are provided, comprising administering to the subject (e.g., mammalian subject; human subject) such a composition.

In some embodiments of the disclosure, the prebiotic formulation comprises one or more prebiotic compounds selected from a fructo-oligosaccharide, a short-chain fructo-oligosaccharide, inulin, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber.

In some embodiments, the prebiotic compound is gel-based. In some embodiments, the prebiotic compound is gel-based inulin.

In some embodiments of the disclosure, the one or more probiotic cells are able to alter the gut microbiome of a subject upon administration to the subject.

In some embodiments, the composition is configured for oral administration to a subject.

In some embodiments, the one or more probiotic cells comprise beneficial bacteria. In some embodiments, the beneficial bacteria comprises one or more of: Saccharomyces cereviseae, Bacillus coagulans, Bacillus licheniformis, Bacillus subtilis, Bifidobacterium angulatum, Bifidobacterium animalis, Bifidobacterium bifidum, Bifidobacterium breve, Bifidobacterium infantis, Bifidobacterium lactis, Bifidobacterium longum, Enterococcus faecium, Enterococcus faecalis, Lactobacillus acidophilus, Lactobacillus amylovorus, Lactobacillus alimentarius, Lactobacillus bulgaricus, Lactobacillus casei subsp. casei, Lactobacillus casei Shirota, Lactobacillus curvatus, Lactobacillus delbrueckii subsp. lactis, Lactobacillus fermentum, Lactobacillus farciminus, Lactobacillus gasseri, Lactobacillus helveticus, Lactobacillus johnsonii, Lactobacillus lacti, Lactobacillus paracasei, Lactobacillus pentosaceus, Lactobacillus plantarum, Lactobacillus reuteri, Lactobacillus rhamnosus (Lactobacillus GG), Lactobacillus sake, Lactobacillus salivarius, Lactococcus lactis, Lactobacillus thermotolerans, Lactobacillus mucosae, Micrococcus varians, Pediococcus acidilactici, Pediococcus pentosaceus, Pediococcus acidilactici, Pediococcus halophilus, Streptococcus faecalis, Streptococcus thermophilus, Staphylococcus carnosus, and Staphylococcus xylosus.

In some embodiments, the composition is a sugar-coated tablet, gel capsule, gel, emulsion, tablet, wafer capsule, hydrogel, nanofiber gel, electrospun fiber, food bar, confectionery, fermented milk, fermented cheese, chewing gum, powder or toothpaste.

In certain embodiments, the present disclosure provides a composition comprising a gel-based prebiotic formulation. In some embodiments, the composition is formulated for oral ingestion. In some embodiments, the composition is a sugar-coated tablet, gel capsule, gel, emulsion, tablet, wafer capsule, hydrogel, nanofiber gel, electrospun fiber, food bar, confectionery, fermented milk, fermented cheese, chewing gum, powder or toothpaste.

In some embodiments of the disclosure, the prebiotic formulation comprises one or more prebiotic compounds selected from a fructo-oligosaccharide, a short-chain fructo-oligosaccharide, inulin, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, glycerol, a polydextrose, a D-tagatose, an acacia fiber, carob, an oat, and a citrus fiber.

In some embodiments of the disclosure, the gel-based prebiotic formulation is associated with one or more probiotic organisms. In some embodiments, the one or more probiotic organisms are chosen from Lactobacillus species and Bifidobacterium species. In some embodiments, the gel-based prebiotic formulation is associated with one or more bacteriophages specific to Bordetella, Borrelia, Brucella, Campylobacter, Chlamydia and Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema, Vibrio and Yersinia. In some embodiments, the one or more probiotic organisms are selected from L. acidophilus, L. amylovorus, L. brevis, L. casei, L. casei subsp. rhamnosus (Lactobacillus GG), L. caucasicus, L. crispatus, L. delbrueckii subsp. bulgaricus (L. bulgaricus), L. fermentum (L. fermenti), L. gasseri, L. helveticus, L. johnsonii, L. lactis, L. leichmannii, L. paracasei, L. plantarum, L. reuteri, or L. rhamnosus.

According to the disclosure, such methods are not limited to a particular manner of administering the agent capable of elevating the richness and diversity of a subject's gut microbiome. According to the invention, the agent is administered orally (e.g., by oral gavage). However, according to the disclosure, administration can be by any suitable route of administration including buccal, dental, endocervical, intramuscular, inhalation, intracranial, intralymphatic, intramuscular, intraocular, intraperitoneal, intrapleural, intrathecal, intratracheal, intrauterine, intravascular, intravenous, intravesical, intranasal, ophthalmic, otic, biliary perfusion, cardiac perfusion, priodontal, rectal, spinal subcutaneous, sublingual, topical, intravaginal, transermal, ureteral, or urethral. Dosage forms can be aerosol including metered aerosol, chewable bar, capsule, capsule containing coated pellets, capsule containing delayed release pellets, capsule containing extended release pellets, concentrate, cream, augmented cream, suppository cream, disc, dressing, elixer, emulsion, enema, extended release fiber, extended release film, gas, gel, metered gel, granule, delayed release granule, effervescent granule, chewing gum, implant, inhalant, injectable, injectable lipid complex, injectable liposomes, insert, extended release insert, intrauterine device, jelly, liquid, extended release liquid, lotion, augmented lotion, shampoo lotion, oil, ointment, augmented ointment, paste, pastille, pellet, powder, extended release powder, metered powder, ring, shampoo, soap solution, solution for slush, solution/drops, concentrate solution, gel forming solution/drops, sponge, spray, metered spray, suppository, suspension, suspension/drops, extended release suspension, swab, syrup, tablet, chewable tablet, tablet containing coated particles, delayed release tablet, dispersible tablet, effervescent tablet, extended release tablet, orally disintegrating tablet, tampon, tape or troche/lozenge.

Intraocular administration can include administration by injection including intravitreal injection, by eyedrops and by trans-scleral delivery.

Administration can also be by inclusion in the diet of the mammal such as in a functional food for humans or companion animals.

As noted, according to the invention, agents capable of elevating the richness and diversity of a subject's gut microbiome are to be administered orally. Such formulations are preferably encapsulated and formulated with suitable carriers in solid dosage forms. Some examples of suitable carriers, excipients, and diluents include lactose, dextrose, sucrose, sorbitol, mannitol, starches, gum acacia, calcium phosphate, alginates, calcium silicate, microcrystalline cellulose, polyvinylpyrrolidone, cellulose, gelatin, syrup, methylcellulose, methyl- and propylhydroxybenzoates, talc, magnesium, stearate, water, mineral oil, and the like. The formulations can additionally include lubricating agents, wetting agents, emulsifying and suspending agents, preserving agents, sweetening agents or flavoring agents. The compositions may be formulated such as to provide rapid, sustained, or delayed release of the active ingredients after administration to the patient by employing procedures well known in the art. The formulations can also contain substances that diminish proteolytic degradation and promote absorption such as, for example, surface-active agents.

The specific dose can be calculated according to the approximate body weight or body surface area of the patient or the volume of body space to be occupied. The dose will also depend upon the particular route of administration selected. Further refinement of the calculations necessary to determine the appropriate dosage for treatment is routinely made by those of ordinary skill in the art. Such calculations can be made without undue experimentation by one skilled in the art in light of the activity in assay preparations such as has been described elsewhere for certain compounds (see for example, Howitz et al., Nature 425:191-196, 2003 and supplementary information that accompanies the paper). Exact dosages can be determined in conjunction with standard dose-response studies. It will be understood that the amount of the composition actually administered will be determined by a practitioner, in the light of the relevant circumstances including the condition or conditions to be treated, the choice of composition to be administered, the age, weight, and response of the individual patient, the severity of the patient's symptoms, and the chosen route of administration.

The present disclosure also provides kits comprising an agent capable of elevating the richness and diversity of the subject's gut microbiome (e.g., a fiber based pre-biotic), and one or more of a vaccine (e.g., a cancer vaccine) (e.g., a vaccine for treating and/or protecting from infectious pathogens), and a cancer immunotherapy (e.g., an ICI inhibitor). The kits may optionally contain other therapeutic agents.

### EXPERIMENTAL

The following examples are provided to demonstrate and further illustrate certain preferred embodiments of the present invention and are not to be construed as limiting the scope thereof, which is as defined in the claims. Examples relating to inulin gel in combination with a cancer immunotherapy or vaccine are according to the invention, in particular examples V-VII, XII and XIII. The other examples are relevant for understanding the invention.

### Example I.

This example demonstrates efficacy improvement for immune checkpoint blockers with prebiotics in a prophylatic setting.

Among the FDA's list, five candidate materials were selected for the initial screening. Melatonin, found in pineal gland and gastrointestinal tracta, regulates sleep cycle and circadian rhythm. Epigallocatechin gallate (EGCG) is a natural antioxidant in plants. Fucoidan, oligofructose and inulin are plant polysaccharides that are widely used in food products and diet supplements. These candidate materials were first tested for their ability to improve the antitumor efficacy of α-PD-1 therapy in a prophylactic manner. WT Balb/c mice were treated with these materials via oral gavage one week before tumor inoculation **(****Fig. 1A****).** All these agents combined with α-PD-1 therapy exhibited stronger anti-tumor efficacy, compared with α-PD-1 alone, and inulin exhibited the best efficacy **(****Fig. 1B and C****).** Inulin combined with α-PD-1 prolonged the survival of animals with 60% mice still surviving on day 50 **(****Fig. 1D****).**

The effect of these materials were next tested on systemic immune responses. The surrogate marker of tumor specific antigen, MHC-I minimal epitope of CT-26 gp70 (AH1) (H-2L^{d}-restricted SPSYVYHQF (SEQ ID NO.: 1)), was employed to quantitate the frequency of AH1-specific CD8⁺ T cells among peripheral blood mononuclear cells (PBMCs). Mice orally gavaged with inulin and melatonin combined with α-PD-1 IgG induced 1.7-fold and 1.8-fold greater frequencies of AH1-specific CD8⁺ T cells, compared with α-PD-1 treatment alone **(****Fig. 1E and F****).**

### Example II.

This example describes improvement in the efficacy of immune checkpoint blockers with prebiotics in a therapeutic setting.

Having identified inulin as a promising candidate, these agents were next tested in a therapeutic setting. After Balb/c mice were inoculated with CT-26 cells, tumor-bearing mice were treated with oral gavage starting day 7 **(****Fig. 2A****).** Consistent with the prophylactic treatment, inulin combined with α-PD-1 IgG therapy exhibited the strongest anti-tumor efficacy **(****Fig. 2B and C****)** with an extended animal survival **(****Fig. 2D****).** Inulin also elicited significantly higher AH1-specific CD8⁺ T cells among PBMCs on day 18 and 24 **(****Fig. 2E-G****).**

### Example III.

This example describes dosage effects of inulin and melanotinin.

The dosage effect of inulin and melatonin were next examined, which were the top 2 candidates identified above. Reducing the dosage of inulin and melatonin by half (i.e., 60 mg for inulin and 50 mg/Kg for melatonin) did not compromise their combination efficacy with α-PD-1 against tumor growth **(****Fig. 3A, B****),** suggesting a relatively wide therapeutic window of inulin and melatonin. Similar results were also found in the frequency of AH1-specific CD8⁺ T cells among PBMCs **(****Fig. 3C, D****).** Since tumor-infiltrating lymphocytes play pivotal roles in the outcome of immunotherapy, T lymphocytes in the tumor microenvironment were examined. Inulin or melatonin treatment combined with α-PD-1 IgG significantly enhanced intratumoral infiltration of T cells, compared α-PD-1 IgG alone **(****Fig. 3E-H****).**

### Example IV.

This example describes changes in the gut microbiome.

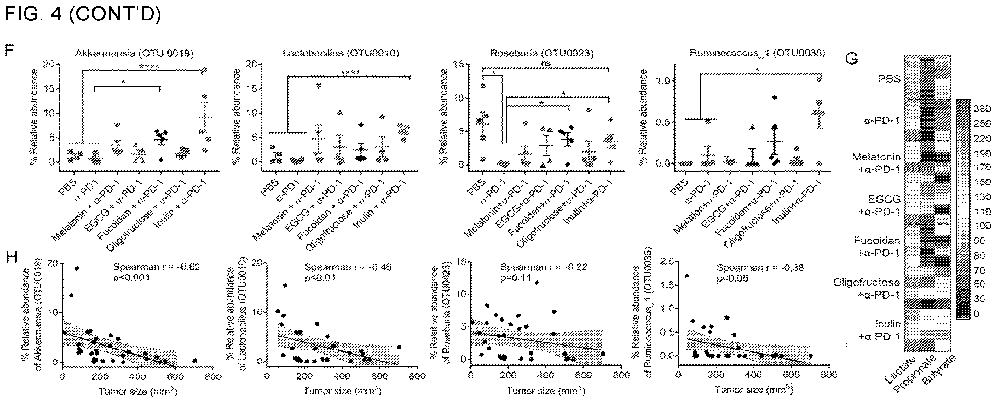

The composition of the gut microbiome during the treatment was examined via 16S rDNA sequencing analysis. Oral gavage of these materials increased the operational taxonomic unit (OUT, **Fig. 4A****)** as well as inverse Simpson diversity value **(****Fig. 4B****),** compared with PBS group, indicating that the treatments elevated the richness and diversity of gut microbiome. Nonmetric multidimensional scaling analysis (NMDS) clearly showed that oral gavage of these materials led to a distinct clustering of microbial community structure compared with PBS or free α-PD-1, especially for inulin **(****Fig. 4C****).** Further analysis in the family/genus level showed that oral administration of inulin, followed by α-PD-1 treatment increased the relative abundance of Akkermansia, Lactobacillus, Ruminococcus, and Butyricicoccus post, compared with α-PD-1 alone or PBS group **(****Fig. 4D-F****,** **Fig. 5A****).** Recent studies have shown that Lactobacillus and Akkermansia can improve the anti-tumor efficacy of immune checkpoint blockade in tumor-bearing mice, while Butyricicoccus and Ruminococcus are known to generate short chain fatty acids (SCFAs) including butyrate as metabolites. Microbiome metabolite SCFAs, especially butyrate, are known to improve immune response as well as inhibit inflammation. Surprisingly, it was also found that free α-PD-1 treatment dramatically decreased the abundance of Roseburia (another crucial butyrate-producing bacteria) to almost 0%, compared to PBS group **(****Fig. 4F****).** Oral gavage of the candidate agents, especially inulin, increased the abundance of Roseburia in the gut. Analysis of SCFAs in fecal pellets further confirmed that oral gavage of inulin increased the concentration of lactate, propionate and butyrate **(****Fig. 4G****).** In contrast, free α-PD-1 group had lower propionate and butyrate, compared with the PBS group. Furthermore, the fitting curves and Spearman's correlation coefficients analysis showed that the tumor sizes were negatively correlated with the relative abundance of Akkermansia, Lactobacillus, Roseburia, Ruminococcus, and Butyricicoccus **(****Fig. 4H****),** suggesting beneficial roles of these commensal bacteria for cancer treatment. NMDS and microbiome composition results further clearly demonstrated that free inulin and melatonin, but not α-PD-1, played the dominated role in manipulating the microbial community structure during the combined therapy with α-PD-1 **(****Fig. 5****).**

### Example V.

This example demonstrates that inulin gel further improves the efficacy of immune checkpoint blockade.

Given that inulin showed the best efficacy among these candidate materials with α-PD-1 and the reduced dosage (60 mg/dosage) didn't compromise the efficacy obviously, a 60 mg/dosage was chosen for inulin in the following study. Inulin gel via was prepared via a heating-cooling method, which ensured a large scale production with ease **(****Fig 6A****).** This gel is injectable for oral gavage **(****Fig. 7A****).** Scanning electron microscope (SEM) revealed that inulin gel exhibited fabric-like surface morphology **(****Fig. 6A****).** Reduced sugar assay verified stability of inulin gel at stomach-like acidic environment for 2 h **(****Fig. 7B****).** The antitumor effect of combining α-PD-1 was compared with either free inulin or inulin gel. Strikingly, inulin gel exhibited significantly higher tumor inhibition efficacy than free inulin **(****Fig. 7B****).** The survival rate in inulin gel group was signifcantly prolonged, and 60% mice completely eradicated established tumors **(****Fig 6C****).** These survivors were protected against tumor re-challenge with 1.5×10⁵ CT26 tumor cells **(****Fig 6D****),** demonstrating long-term immunity against tumor replase. Tetramer staining assay revealed that the frequency of AH1-specific CD8⁺ T cells among PBMCs was significantly elavated after gavage of inulin gel, compared with free inulin **(****Fig 6E****).** Meanwhile, the amount of tumor infiltrating CD45⁺CD8⁺ T cells, CD45⁺CD4⁺ T cells, as well as matured DC cells (CD86⁺CD11C⁺) were significantly increased **(****Fig. 6F****).** Note that these tumor infiltrating CD8⁺ T cells in inulin gel group exhibited better functionality against tumor in terms of improved AH1-tetramer⁺ and decreased PD-1⁺ biomarkers on cell surfaces, compared with inulin group. IFN-γ ELISPOT assay in spleen further revealed that inulin gel combined with α-PD-1 elicited robust IFN-γ expression among CD8⁺ T cells **(****Fig 6G and H****),** which was 3.5 and 4.6-fold higher than the inulin+α-PD-1 group and α-PD-1 group, respectively. Besides, the administration of α-CD8 depletion antibody completely abrogated the antitumor efficacy of inulin gel + α-PD-1. In sharp contrast, administration of α-Asialo GM1 or α-CD4 antibody did not affect the therapeutic efficacy of inulin gel + α-PD-1 therapy **(****Fig 6I**), revealing that CD8⁺ T cells, but not CD4⁺ T cells or NK cells, are the key lymphocytes for tumor inhibition and eradication.

### Example VI.

This example demonstrates that inulin gel with improved retention in colon alters the gut microbiome.

FITC-inulin was employed to synthesize FITC-labeled inulin gel. In vivo gastrointestinal retetion imaging showed that the inulin gel group exhibited improved accumulation and retention in cecum and colon at 4.5 h post gavage, compared with free inulin group **(****Fig 8A,B****).** Fecal pellets were collected at preset time points post gavage of FITC-inulin or FITC-inulin gel and confirmed increased retention of FITC-inulin gel, compared with inulin **(****Fig 8C****).** These results were confirmed using an inulin detection kit, and the area under curve value clearly domonstrated the better retention of inulin gel in colon, compared with inulin **(****Fig 8D****).**

This prolonged retention of inulin gel resulted in increased OTU and inverse Simpson diversity values **(****Fig 8E****).** NMDS result and microbiome analysis in family level showed that there was a shift in microbial community structure to some extent between inulin and inulin gel groups **(****Fig 8F****).** The microbiome composition in genera level revealed that the relative abundance of Akkermansia, Ruminococcus, and Roseburia were significantly increased post gavage of inulin gel, compared with inulin group **(****Fig 8G,H****),** while the abundance of Lactobacillus and Butyricicoccus were comparable between these two groups.

To confirm the role of gut microbiome in antitumor efficacy, mice in specific pathogenfree (SPF) housing condition were given broad-spectrum antibiotics (ampicillin + colistin + streptomycin) for 7 days before tumor inoculation and continuous antibiotics treatment from day 5 post tumor inoculation. Broad-spectrum antibiotics treatment dramatically compromised the antitumor effects of inulin gel combined with α-PD-1 **(****Fig. 8I****),** demonstrating the crucial role of gut microbiome in the combination inulin gel plus α-PD-1 therapy.

### Example VII.

This example demonstrates the therapeutic efficacy of inulin gel in a second tumor model.

The inulin gel system was evaluated in MC38 tumor model using C57BL/6 mice as another mouse strain **(****Fig 9A****).** Previous study showed that MC38 cells had mutated neo-epitope within the Adpgk protein (ASMTNRELM (SEQ ID NO.: 2)→ASMTNMELM (SEQ ID NO.: 3) mutation) in the context of H-2Db molecules. Inulin gel combined with α-PD-1 induced higher IFN-γ expression in splenoic CD8⁺ T cells via IFN-γ ELISPOT assay **(****Fig 9B,C****),** compared with either α-PD-1, inulin gel, or free inulin + α-PD-1. As a result, inulin gel significantly slowed down the tumor growth and prolonged the survival of tumor-bearing mice **(****Fig 9D,E****).**

### Example VIII.

This example demonstrates the safety of inulin gel plus α-PD-1 therapy.

The safety of inulin gel system was evaluated. Mice treated with inuline gel plus α-PD-1 therapy did not exhibit any changes in the complete blood counts and blood chemistry panels, compared with PBS treated mice. In addition, H&E staining of major organs did not show any signs of overt inflammation or tissue damage after inuline gel plus α-PD-1, indicating the safety of the combination therapy.

### Example IX.

This example provides the materials and methods utilized in Examples I-VIII.

### Inulin hydrogel preparation and characterization

300 mg of inulin (Sigma-Aldrich) was dissolved in 1.2 mL DI water. Then inulin solution was heated at 70°C for 5 min and kept at room temperature for 12 h to obtain inulin gel. For SEM observation, inulin gel was flash-frozen in liquid nitrogen and lyophilized. The samples were then sputter-coated with gold for 30s. The samples were visualized with MIRA3 TESCAN (voltage 15 kV). To simulate the potential degradation of inulin gel in stomach acid environment, inulin gel was incubated at strong acidic water (pH 2) for 2 or 24 h with gentle agitation. Samples were centrifuged and supernatant was collected. The dinitrosalicylic acid (DNS) method (Miller, 1959) was used for the quantitative analysis of the reducing sugar in supernatant. The intensity of developed color was measured at 540 nm using a microplate reader.

### In vivo cancer immunotherapy

Mice were cared for under the guidelines from federal, state and local. All experimental animal procedures were approved by the University Committee on Use and Care of Animals (UCUCA) at University of Michigan, Ann Arbor. Female C57BL/6 or BALB/c mice of 6-8-week old were obtained from Jackson Laboratory. BALB/c mice were inoculated with 1.5 × 10⁵ CT26 cells per mouse, while C57BL/6 mice were inoculated with 1.2 × 10⁶ MC38 cells per mouse on the right flank by subcutaneous injection on day 0. Tumor-bearing mice were randomly assigned to different groups on day 5. For prophylactic antitumor study, mice were received oral gavage with melatonin (100 mg/Kg bodyweight/dosage), EGCG (100 mg/Kg bodyweight /dosage), fucoidan (200 mg/Kg bodyweight /dosage), oligofructose (120 mg/dosage), or inulin (120 mg/dosage) three times for one week prior to tumor inoculation. After tumor inoculation, mice received oral gavage three times in the first week, followed by five times per week. Mice were injected *i.p.* with α-PD-1 antibody (100 µg/dosage) on days 10, 14, 18 and 22 post tumor inoculation. For the therapeutic therapy, mice were inoculated with tumor cells on day 0 as indicated above and treated with the indicated samples by oral gavage starting day 7 post tumor inoculation. Mice received oral gavage of the indicated samples (same dosage with the prophylactic antitumor study) five times per week while α-PD-1 antibody (100 µg/dose) was injected on days 11, 15, 19 and 23 post-tumor inoculation. Tumor size was measured at preset time, and tumor volume was calculated as length × width². For anti-tumor studies with inulin gel, the dosage of inulin gel or inulin was 60 mg/dosage. For the survival study, tumor-bearing mice were euthanized when the tumor size reached 1.5 cm in diameter or when animals became moribund with severe weight loss (> 20%) or had tumor ulceration exceeding 50% of the tumor volume. In the antibody depletion studies, CT26 tumor-bearing mice received oral gavage with inulin gel (60 mg/dosage) and α-PD-1 treatment as indicated above. CD8 T-cells, CD4 T-cells, and NK cells were depleted by i.p. administration days 8, 11 and 16 with 200 µg/dosage of anti-CD8 (Bioxcell, clone 2.43, #BP0061), anti-CD4 (Bioxcell, clone GK1.5, #BP0003-1), or anti-Asialo GM1 (Wako chemicals USA, Inc, #986-10001), respectively.

### Tetramer analysis in PBMCs

Peripheral blood mononuclear cells (PBMCs) were collected at preset times to analyze tumor antigen-specific CD8⁺ T cells in the systemic circulation. Red blood cells were lysed, and the remaining cells were blocked with anti-CD16/32 antibody for 10 min and stained with peptide-MHC tetramer, including H-2L^{d}-restricted SPSYVYHQF (SEQ ID NO.: 1) or H-2D^{b}-restricted ASMTNMELM (SEQ ID NO.: 3). Both the tetramers were provided by NIH Tetramer Core Facility. Cells were also stained anti-CD8-APC (BD Biosciences, #553035) and DAPI for flow cytometric analysis.

### Tumor microenvironment analysis

After treatment with various samples, tumor tissues were excised at preset time points for analysis of tumor-infiltrating T cells. Tumor tissues were excised, cut into small pieces, and incubated with collagenase type IV (1 mg/ml) and of DNase I (0.1 mg/ml) with gentle shaking. After 30 min, cell suspension was filtered through a 70-µm strainer. Cells were washed with FACS buffer and blocked with anti-CD16/32 antibody. Cells were then stained with various antibodies (CD4 Monoclonal Antibody (GK1.5)-PE (eBioscience), APC-Anti-CD45 Rat Monoclonal Antibody (clone: 30-F11), FITC-Anti-CD45 Rat Monoclonal Antibody (clone: 30-F11), FITC-Rat Anti-Mouse CD8a Clone 53-6.7 (BD Biosciences), PE labeled H-2L^{d}-restricted SPSYVYHQF (SEQ ID NO.: 1), FITC-Anti-CD86 Rat Monoclonal Antibody (clone: GL-1, BioLegend), PE-CD11c Armenian Hamster anti Mouse (Clone: N418, eBioscience), PE-Cy7-Anti-mouse PD-1 (clone: 29F.1A12, BioLegend), FITC-CD44 Rat anti-Human/Mouse (clone: IM7, eBioscience), PE-Cy7-CD62L Monoclonal Antibody (MEL-14) (eBioscience), PE-Tcf1/Tcf7 (C63D9) Rabbit mAb (Cell Signaling Technology, #14456), PE-Cy7-Anti-mouse CD86 (Clone: GL1, BD Bioscience), FITC anti-mouse/human CD11b (M1/70, BioLegend), PE-anti-mouse F4/80 (BM8, BioLegend), or APC anti-mouse CD206 (MMR, BioLegend)). The cells were washed and stained with DAPI, or 7-AAD, or efluor 450 for flow cytometric analysis.

### Gut microbiome analysis

Tumor-bearing mice received treatments as indicated above. On day 20, the fecal pellets were collected and stored at -80°C before the gut microbial analysis. Both microbiome DNA isolation and 16S rRNA Sequencing were completed by The University of Michigan Microbial Systems Molecular Biology Laboratory. Isolation of microbial DNA from mice fecal samples was performed using Qiagen MagAttract Power Microbiome kit. The V4 region of the 16S rRNA-encoding gene was amplified from the extracted DNA using the barcoded dual-index primers developed by Kozich et al. (1). Briefly, barcoded dual-index primers specific to the V4 region of the 16S rRNA gene amplified the DNA. The conditions for PCR were as follows: 2 min at 95°C, 30 cycles of 95°C for 20 s, 55°C for 15 s, 72°C for 5 min, and 72°C for 10 min. The size of the amplicon library (~399 bp) was confirmed by Agilent Bioanalyzer. Pooled amplicon library was then sequenced on the Illumina MiSeq platform using the 500 cycle MiSeq V2 Reagent kit (catalog no. MS-102-2003) according to the manufacturer's instructions with modifications of the primer set with custom read 1/read 2 and index primers added to the reagent cartridge. The "Preparing Libraries for Sequencing on the MiSeq" (part 15039740, Rev. D) protocol was used to prepare libraries with a final load concentration of 5.5 pM and spiked with 15% PhiX to create diversity within the run. The microbial 16S rRNA gene sequencing data from mice fecal collections were processed by Mothur. Silva reference files (release 132) were used to align sequences, and the open reference OTU picking protocol was used at 97% sequence identity.

### Fecal pH and SCFAs measurement

Fresh fecal pellets of mice were collected and weighted on day 20 post tumor inoculation and treatments. Fecal pellets were homogenized in DI water and centrifuged at 3000 X g for 5 min. The pH of the supernatant was detected by pH meter. For SCFAs measurement, SCFAs were extracted from the fecal pellets using Milli-Q water. The solution was centrifuged for 5 min at 10000 X g (4°C) to pellet bacteria and other solids. The supernatant was collected, and SCFAs were measured via ion chromatography in University of Michigan Biological Station. The concentration of SCFAs was determined using an external standard method.

### IFN-γ ELISPOT assay

CT26 tumor-bearing BALB/c mice received the indicated samples via oral gavage for five times per week starting day 7 of tumor inoculation. The animals were also administered i.p. with α-PD-1 antibody (100 µg) on days 11, 15, and 19. On day 23, the animals were euthanized, and their spleens were analyzed for anti-tumor T-cell responses using IFN-γ ELISPOT assay. ELISPOT plate was coated with capture antibody for 24 h and blocked with DMEM + 10% FBS for 2 h. These splenocytes were added to 96-well plate with a density of 2 × 10⁵ alive cells/well. Meanwhile, AH1 peptide (SPSYVYHQF (SEQ ID NO.: 1), 20 µg/mL) was added to stimulate splenocytes. Ionomycin and PMA were employed as the positive control. After 18 h, IFN-γ spots were detected with biotinylated detection antibody, followed by streptavidin-HRP and AEC substrate kit. The number of IFN-γ spots were measured in the Cancer Center Immunology Core of University of Michigan. For IFN-γ ELISPOT assay in MC38 tumor-bearing C57BL/6 mice was conducted in a similar way. PBMCs obtained on day 20 were used for IFN-γ ELISPOT assay.

### Inulin hydrogel retention in the gastrointestinal system

Retention of inulin gel in the gastrointestinal system was measured using FITC-inulin gel. To prepare FITC-tagged inulin hydrogel, 47.5 mg inulin and 12.5 mg FITC-inulin (Sigma-Aldrich) were mixed in 200 µL DI water. For in vivo imaging of inulin, mice received oral gavage with FITC-tagged inulin or inulin gel and euthanized at preset time points. The stomach, small intestine, cecum, colon and rectum were harvested and washed with PBS before fluorescence imaging with the IVIS optical imaging system. For detection of inulin content in fecal pellets, FITC-tagged inulin or inulin gel was orally gavaged into mice, and the fecal pellets were collected at preset time points. The fecal pellets were weighted and resuspended in DI water. The sample solution was then centrifuged, and the fluorescence intensity of supernatants was measured using a microplate reader. The fluorescence intensity was normalized to 1 mg/mL fecal sample. For detection of inulin content in colon, mice were gavaged with inulin or inulin gel (60 mg/dose) and euthanized at preset time points post gavage. The fecal pellets in colon was harvested, weighted, homogenized, and centrifuged. The supernatants were diluted 20 times and used for detection of inulin. Inulin was detected via PicoProbe^{™} Inulin Assay Kit (BioVision) according to the manufacturer's protocol.

### Antibiotic water treatment

Mice received sterile drinking water containing antibiotics for one week before tumor inoculation. After tumor inoculation, the animals received normal drinking water for 5 days to avoid the direct effect of antibiotics on tumor engraftment and formation. After 5 days, tumor-bearing mice received drinking water with antibiotics for the reminder of the studies. The antibiotic drinking water containing ampicillin (0.3 mg/ml), streptomycin (2.5 mg/ml), and colistin (0.3 mg/ml) was replaced twice every week.

### Biosafety evaluation

CT26 tumor-bearing mice were treated with the regimen as described above. On day 29, blood samples were collected for complete blood count (CBC) (including eosinophils, lymphocytes, monocytes, platelets, red blood cells, hemoglobin, mean platelet volume, red cell distribution width (RDW) and mean corpuscular hemoglobin (MCH)). Serum samples were used for biochemistry analysis, including alanine transaminase (ALT), aspartate aminotransferase (AST), blood urea nitrogen (BUN), glucose, cholesterol, and creatine phosphokinase (CPK). Mice were euthanized on day 29, and lung, liver, spleen, heart and kidney were collected for H&E staining.

### Statistical analysis

Animal studies were performed after randomization. Data were analyzed by paired or unpaired t-tests or one- or two-way analysis of variance (ANOVA), followed by multiple comparison test with Prism 8.0 (GraphPad Software). p < 0.05 was considered statistically significant.

### EXAMPLE X.

This example demonstrates a method of delivering bacteria and probiotics using inulin-gel formulation.

There is intense research interest for colon-targeted delivery of probiotics for improving health benefits. A new strategy of using prebiotic-based gel formulations for co-delivery of bacteria (probiotics) was developed. As a proof-of-concept, E. coli was used as a model bacteria organism and used inulin-gel formulation described earlier to encapsulate and deliver E. coli.

E. coli expressing GFP was used to assess entrapment and growth of E. coli in inulin-gel formulation. E. coli entrapped in inulin gel demonstrated a typical bacteria growth pattern of four phases (**Figure 10**). The cell density was proportional to the GFP expression before reaching the stationary phase. The cell number stayed constant in stationary phase and collapsed in lag phase due to the exhaustion of nutrient and generation of metabolites, while the fluorescence accumulated gradually. This demonstrated that inulin-gel can be used to entrap E. coli and maintain its viability.

The growth of E. coli in culture dishes was compared versus in inulin-gel. E. coli showed comparable fluorescence intensity between inulin gel-entrapped group and PBS control group (i.e. culture dish). The inulin gel emitted negligible autofluorescence at the wave length of GFP.

When viability of E. coli was compared by CFU counting, it was observed that E. coli entrapped in inulin gel exhibited increased viability (Table 1). The calculated CFU of inulin gel group was 3-fold higher than the number acquired in PBS. This demonstrates that inulin gel can entrap and promote the growth of bacteria. These results, combined with the results shown in prior examples, indicate that inulin gel designed for colon retention can be used for entrapping bacteria (probiotics) and delivering them to colon tissues.

**Table 1. Cell Viability determined by CFU counting after 24hrs incubation in PBS and encapsulation in inulin gel respectively.**

| E. coli@PBS | E. coli@Inulin Gel |
|---|---|
| 1.61×10⁸CFU/mL | 5.25×10⁸ CFU/mL |

### Example XI.

This example describes the materials and methods utilized in Example X.

E. coli Culture and CFU Counting E. coli was cultured in Tryptic Soy Broth (TSB) media supplemented with 100 µg/mL ampicillin, 37°C, 200 rpm, overnight. Optical density at 600nm was measured. 10-fold serial dilution was applied to cell culture and dilutions were spread onto TSB agar plate, culturing for 12 hrs, 37°C. CFU was calculated through numbers of each dilution factor.

Inulin Gel Preparation and E. coli Encapsulation Inulin (330 mg/mL) was suspended in sterile PBS buffer using vortex. Suspension was heated to 70°C for 5 mins and then cooled down for 10 mins under room temperature. E. coli culture of 7.5 × 10⁹ CFU/mL density was added to the cooled suspension in a ratio of E.coli : Inulin = 1:9, acquiring the E. coli gel with final concentration of 7.5×10⁸ CFU@300 mg/mL.

### Example XII.

Figures 1-10 showed results generated in BABL/c mice obtained from Jackson Laboratory. As mice from different animal vendors are known to have distinct gut microbiome, experiments were conducted that sought to validate the therapeutic efficacy of inulin gel plus α-PD-1 combo-therapy in mice obtained from other animal vendors. BABL/c mice obtained from Charles River and Taconic Farm were inoculated with CT26 tumor cells and treated with inulin gel plus α-PD-1 combo-therapy as shown in Figure 13A. Inulin gel plus α-PD-1 combo-therapy exerted anti-tumor efficacy in BABL/c mice obtained from Charles River and Taconic Farm (Figure 13B-C), thus showing that the anti-tumor efficacy of the combo-therapy is not vendor-specific effects.

In addition to CT26 tumor model in BABL/c mice, experiments were conducted that sought to validate these results in other tumor models. Inulin gel (60 mg/dose) plus α-PD-1 combo-therapy also generated strong CD8⁺ T cell responses with robust anti-tumor efficacy in C57BL/6 mice (Jackson Laboratory) bearing either MC-38 colon carcinoma or B16F10 melanoma (Figure 13D-E), thus showing utility of this strategy in multiple tumor models in multiple mouse strains.

### Example XIII.

In order to understand the impact of the composition of inulin gel on *in vivo* outcomes, experiments were conducted that synthesized various inulin gel formulations using inulin of various degrees of polymerization (DP).

### Materials and Methods

Inulin samples were purchased from Now Foods, Swanson Health Products, Sigma-Aldrich, and Orafti.

¹H-NMR. Each 5 mg of inulin powder was dissolved in 600 µL D₂O to prepare ¹H-NMR samples. Samples were run on Agilent/Varian 400MHz NMR spectrometer with a 5mm ONE probe with multinuclear capabilities, operated by host software VNMRJ 3.2. The DP was calculated through the ¹H-NMR spectrum as described previously (T. Barkhatova, M. Nazarenko, M. Kozhukhova, I. Khripko, Foods and Raw materials 2015, 3).

MALDI-TOF-MS. Each 10 mg inulin sample was dissolved in 1.00 ml water, 0.25 ml methanol, and 0.25 ml acetonitrile. Then 2 µL sample solution was mixed with the matrix solution, and spotted onto the target plate. The matrix solution was 2,5-DHB, at a concentration of 4 mg/mL, dissolved in 50/50 acetonitrile/water with 0.1% TFA. The samples were run on Bruker Autoflex Speed in linear mode. The instrument was mass calibrated with a mixture on bovine insulin and cytochrome C in a 2,5-DHB matrix.

Preparation of inulin gel. A desired weight of inulin powder was dissolved in 1.0 mL deionized water. Then inulin solution was heated at 70°C for 5 min with 1000 rpm shake and kept at room temperature overnight for gelatinization.

Rheology test. Inulin gel (samples 3 and 5) for rheology test was prepared as described above with a concentration of 300 mg inulin in 1.0 mL deionized water. Inulin (3) suspension was prepared with same concentration but without heating and cooling procedures. Inulin gel (sample 1) was prepared with same gelatinization steps but in a concentration of 700 mg inulin in 1.0 mL deionized water. Measurements were performed at 25°C with MCR702 TwinDrive rheometer manufactured by Anton Paar equipped with parallel PP60 Ti plates with 0.100 mm gap. All tests were conducted at the strain of 1% which was in the range of the linear viscoelastic region of the studied samples. G' represents elastic modulus, G'' represents viscous modulus.

Inulin content in fecal samples. To detect inulin content in fecal samples, mice were oral gavaged with inulin gel (60 mg/dose). And fresh fecal pellets of mice were collected and weighted at preset time points (0, 2.5, 5, 7.5, 10 and 12.5h) post gavage. Each fecal pellet was homogenized in 800 µL DI water and centrifuged at 20000 RCF for 10 min. Then the supernatants were diluted 25 times for quantifying the inulin content with PicoProbe^{™} Inulin Assay Kit (BioVision) according to the manufacturer's instructions.

*In vivo* study. CT26 tumor-bearing BALB/c mice (female, Jackson Laboratory, aged 6-8 weeks) were randomly assigned to different groups for treatments. Mice were gavaged with inulin gel (60 mg/dose) four times in every five days, and α-PD-1 antibody (100 µg/dose) was administrated via intraperitoneal injection once every four days. Starting from day 5 post tumor inoculation, the tumor volume and mouse body weight were measured every other day. Tumor volume was calculated as (length × width²)/2.

Statistical analysis. GraphPad Prism 8 was used to analyze the statistical data. The results are expressed as means ± SEM. Differences among groups were analyzed by two-way ANOVA with Bonferroni's multiple comparisons test. The differences were considered significant for p values *P < 0.05, **P < 0.01 and ***P < 0.001.

### Results

Inulin samples with various degrees of polymerization (DP) were obtained from different companies and analyzed by ¹H-NMR (Figure 14). ¹H-NMR spectrum analyses revealed the following (Figure 14). Inulin sample #1 had the average DP≈7, Mw≈1314 Da. Inulin sample #2 had the average DP≈10, Mw≈1800 Da. Inulin sample #3 had the average DP≈23, Mw≈3906 Da. Inulin sample #4 had the average DP≈26, Mw≈4392 Da. Inulin sample #5 had the average DP≈28, Mw≈4716 Da.

Furthermore, MALDI-TOF-MS analyses revealed that inulin samples #1, #2, #3, #4 and #5 had molar mass between 990 and 6336 Da (Figure 15). However, the increase in intensity of peaks around 990-2124 Da suggested prevalence of lower DP inulin chain between 5 and 12. It is likely that the inulin chains with low DP are shorter and easier to be excited to be detected, while the long inulin chains with higher DP are tightly entangled with each other and are less likely to be excited off the substrate leading to lower intensity of signal MALDI-TOF-MS analyses.

Experiments were next conducted that examined inulin gel formation using these inulin samples with various DPs. As shown in Figure 16, inulin samples with DP 7 and DP 10 (samples 1 and 2) were more soluble in water before heating, compared with inulin samples with higher DP (samples 3-5). For inulin gel formation under the same condition, much higher concentration (700 mg/g water) of inulin was needed for inulin sample 1 with DP 7, compared with other four inulin samples with higher DPs. Inulin sample 5 with the highest DP 28 was able for form inulin gel with even inulin concentration as low as 100 mg/g in water. These results showed that inulin with higher DP was less soluble in water and requires less concentration for forming inulin gel.

The DP of inulin also has impact on rheological properties of the gel. In Figure 17, with the rise of DP from 7 (sample 1) to 28 (sample 5), both elastic modulus (G') and viscous modulus (G''), increased by ~10-fold. Inulin sample 3 exhibited G' and G'' at intermediate values compared with inulin sample 1 and 5. Compared with free inulin solution, all three inulin gels formed with inulin sample 1, 3, and 5 had greater G' and G" values. These results indicated that these samples were more solid-like rather than liquid, confirming successful inulin gel formation.

Experiments were next conducted that evaluated the colon retention behavior of various inulin gel formulations *in vivo.* Naïve mice were orally gavaged with inulin gel with various DPs, and fecal samples were analyzed for inulin content over time. As shown in Figure 18, inulin gels with lower DPs had an earlier peak time, with 6.17 h post gavage for inulin sample 1 and 5.38 h post gavage for inulin sample 2. And for inulin gels with higher DPs, longer peak time was observed, with 7.73 h for inulin sample 3 and 10.25 h for inulin sample 5. Furthermore, the relative total inulin content in feces gradually decreased with the rise of the DP regarding the area under the curves. These indicated that inulin gel with higher DP prolonged the retention time of inulin in the gastrointestinal tract, which may further increase the utilization of inulin by the gut microbiota.

Experiments were next conducted that assessed the therapeutic efficacy of various inulin gel formulations, namely inulin gel formed with DP 7, 10, 23, and 28. Balb/c mice bearing s.c. flank CT26 tumors were treated with oral gavage of DP-variable inulin gels (60 mg/dose) plus intraperitoneal administration of α-PD-1 (100 ug/dose). Tumor volume and mouse body weight were recorded every other day. Whereas α-PD-1 alone treatment group exhibited a modest antitumor efficacy, α-PD-1 treatment combined with inulin gel formed with either DP 10 or DP 23 exhibited improved anti-tumor efficacy (Figure 19). Notably, inulin gel with DP 28 exhibited markedly enhanced anti-tumor efficacy, compared with inulin gel with DP 10 or DP23 (Figure 19). It was also observed that inulin gel with low DP of 7 seemed to decrease the anti-tumor efficacy of α-PD-1 therapy. These results suggested that the composition of inulin gel had a major role in the anti-tumor efficacy of α-PD-1 therapy and that inulin gel with longer DP exhibited superior an-tumor efficacy, potentially due to prolonged retention time in the gastrointestinal tract as shown in Figure 18. In all animals treated with various inulin gel formulations plus α-PD-1 therapy, no major toxicity or any negative change in animal body weight was observed (Figure 20), indicating safety of the inulin gel plus α-PD-1 combo-therapy.

### Example XIV.

Experiments were next conducted that examined the impact of inulin concentration and temperature on inulin gel formation. Inulin (Sigma) with an average DP 23 was first mixed with water, giving desired concentrations of 15, 20, 25, and 30% w/v. Mixtures were heated to 40, 50, 60, 70 °C respectively for 5 minutes. Gel formation was examined by flipping the Eppendorf tubes after cooling overnight at room temperature. For inulin of given DP 23, higher inulin concentration led to inulin gel formation (Figure 21). Higher temperature applied during the gelation process promote inulin gel formation (Figure 21). These results showed the concentration and temperature dependency of inulin gel formation.

### Example XV.

Experiments were next conducted that examined gel formation using potato starch, another prebiotic natural fiber. The impact of potato starch concentration on gel formation was examined. Food grade potato starch (Bob's Red Mill) was pre-hydrated at desired concentrations of 0.1, 0.5, 2.5, and 5% w/v in water, followed by gentle stirring at 70 °C. Figure 22 shows potato starch gels. Potato starch was instantly gelatinized at concentrations above 5% w/v when the swelling was completed.

### Example XVI.

Experiments were next conducted involving the formation of new fiber gels by combining inulin with other natural dietary fibers or pharmaceutical excipients. Briefly, inulin gels were manufactured by first mixing 23% w/w inulin together with other excipients, including resistant potato starch, pectin, guar gum, bean gum, gelatin, or glycerol. Other natural fibers would provide additional nutrient source for commensal microbiome, while other excipients served as thickening reagents in these formulations. The mixtures were then heated at 70°C for 5 minutes, followed by cooling at room temperature overnight. During the manufacturing processes, both pre-dissolving and dry-blending were used in order to add the excipients, based on individual dissolutions in water. Figure 23 shows the gelation process for #1 inulin gel, #2, inulin gel containing resistant potato starch, #3 inulin gel containing guar gum, #4 inulin gel containing pectin, and #5 inulin gel containing bean gum. The ingredients were first mixed in water (top panel), heated at 70°C for 5 minutes (middle panel), followed by cooling at room temperature overnight, leading to the formation of fiber gels (bottom panel) (Figure 23).

Experiments were next conducted that varied the amount of natural fibers and excipients added to inulin gel and determined the ideal composition for forming fiber gels. As shown in Figure 24, for inulin gels containing inulin content of 23% w/w, they could be reliably added with the following amount of natural fibers or pharmaceutical excipients: 5% potato starch, 2% pectin, 0.5% guar gum, 1.0% bean gum, 5.0% gelatin, or 50% glycerol (w/w). The indicated values are the maximum amount of each ingredient that could be added to 23% w/w inulin gel without disrupting gel formation.

Experiments were next conducted that measured rheological properties of these optimized fiber gels. Rheology measurements were conducted at 25°C with MCR702 TwinDrive Rheometer (Anton Paar, USA) equipped with parallel 40 mm plates with 0.100 mm gap. All frequency sweep measurements were performed at the strain of 1% which is in the range of linear viscoelastic region of the studied formulations. G' represents elastic modulus, G'' represents viscous modulus. Flow curve was obtained as a function of shear rate ranging from to 10⁻¹ to 10² s⁻¹ at 25°C. The reported results are average of at least 3 replications (Figure 25). All tested new fiber gel formulations exhibited weaker gel strength, as shown by the lower values of G' and G'', compared with pure inulin gel. However, inulin gel combined with potato starch demonstrated ~10-fold increase in terms of viscosity.

## Claims

1. An agent capable of elevating the richness and diversity of the human subject's gut microbiome for use in the treatment of a cancer, the treatment comprising, administering to a human subject suffering from cancer
1) a cancer immunotherapy or vaccine, and
2) the agent capable of elevating the richness and diversity of the human subject's gut microbiome,
wherein the agent capable of elevating the richness and diversity of the subject's gut microbiome is administered orally,
wherein the vaccine is a vaccine for treating or preventing cancer,
wherein the cancer is one or more of breast, ovarian, prostate, lung, kidney, gastric, colon, testicular, head and neck, pancreas, brain, melanoma, and other tumors of tissue organs and hematological tumors, such as lymphomas and leukemias, including acute myelogenous leukemia, chronic myelogenous leukemia, chronic lymphocytic leukemia, T cell lymphocytic leukemia, and B cell lymphomas,
wherein the administration of 1) the cancer immunotherapy or a vaccine, and 2) the agent capable of elevating the richness and diversity of the subject's gut microbiome, results in one or more of:
an increased anti-tumor efficacy of the cancer immunotherapy or vaccine,
a stronger immune response (e.g., increased anti-tumor T cell frequency among PBMCs), and
enhanced inhibition of tumor growth,
wherein the agent capable of elevating the richness and diversity of a subject's gut microbiome is a fiber containing prebiotic agent,
wherein the fiber containing prebiotic agent is a gel-based inulin formulation, and
wherein the gel-based inulin formulation has an average degree of polymerization at or higher than 20 and at or less than 47.

2. The agent capable of elevating the richness and diversity of the human subject's gut microbiome for use in the treatment of claim 1, wherein administration of the agent capable of elevating the richness and diversity of the subject's gut microbiome occurs prior to, concurrent with, and/or after administration of the vaccine or cancer immunotherapy.

3. The agent capable of elevating the richness and diversity of the human subject's gut microbiome for use in the treatment of claim 1,
wherein the gel-based inulin formulation has an average degree of polymerization of approximately 28,
wherein the gel-based inulin formulation comprises one or more prebiotic compounds selected from a fructo-oligosaccharide, a short-chain fructo-oligosaccharide, an isomalt-oligosaccharide, a transgalacto-oligosaccharide, a pectin, a xylo-oligosaccharide, a chitosan-oligosaccharide, a beta-glucan, an arable gum modified starch, a resistant potato starch, guar gum, bean gum, gelatin, alginate, glycerol, a polydextrose, a D-tagatose, an acacia fiber, carob, an oat, and a citrus fiber.

4. The agent capable of elevating the richness and diversity of the human subject's gut microbiome for use in the treatment of claim 1, wherein the cancer immunotherapy comprises one or more immune checkpoint inhibitor (ICI) inhibitors,
wherein the one or more ICI inhibitors are capable of binding to, blocking, and/or inhibit the activity of one or more of CTLA-4, PDL1, PDL2, PD1, BTLA, HVEM, TIM3, GAL9, LAG3, VISTA, KIR, 2B4, CD160 and CGEN-15049,
wherein the one or more ICI inhibitors are selected from Tremelimumab (CTLA-4 blocking antibody), anti-OX40, PD-L1 monoclonal Antibody (Anti-B7-H1; MEDI4736), MK-3475 (PD-1 blocker), Nivolumab (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and Yervoy/ipilimumab (anti-CTLA-4 checkpoint inhibitor).

5. The agent capable of elevating the richness and diversity of the human subject's gut microbiome for use in the treatment of claim 1, further comprising administering to the subject one or more chemotherapeutic agents selected from the group consisting of an alkylating agent, an antimetabolite, an anthracycline, an antitumor antibiotic, a monoclonal antibody, a platinum agent, a plant alkaloid, a topoisomerase inhibitor, a vinca alkaloid, a taxane, and an epipodophyllotoxin.

6. The agent capable of elevating the richness and diversity of the human subject's gut microbiome for use in the treatment of claim 1, wherein administration of the agent capable of elevating the richness and diversity of the subject's gut microbiome results in increased relative abundance of Akkermansia, Lactobacillus, Ruminococcus, Roseburia, and Butyricicoccus within the gut microbiome of the subject.

## Patentansprüche

1. Mittel, das in die Lage versetzt, die Reichhaltigkeit und Vielfalt des Darmmikrobioms eines menschlichen Subjekts zur Verwendung bei der Behandlung von Krebs zu steigern, wobei die Behandlung die Verabreichung, an ein menschliches Subjekt, das an Krebs leidet, von
1) einer Krebsimmuntherapie oder einem -Impfstoff umfasst, und
2) das Mittel in der Lage ist, die Vielfalt und die Reichhaltigkeit des menschlichen Darmmikrobioms des menschlichen Subjekts zu steigern,
wobei das Mittel, das in der Lage ist, die Reichhaltigkeit und Vielfalt des Darmmikrobioms des Subjekts zu erhöhen, oral verabreicht wird,
wobei der Impfstoff ein Impfstoff zur Behandlung oder Vorbeugung von Krebs ist,
wobei der Krebs einer oder mehrere ist von Brustkrebs, Eierstockkrebs, Prostatakrebs, Lungenkrebs, Nierenkrebs, Magenkrebs, Darmkrebs, Hodenkrebs, Kopf- und Halskrebs, Bauchspeicheldrüsenkrebs, Hirntumor, Melanom und anderen Tumoren von Gewebeorganen sowie hämatologischen Tumoren wie Lymphome und Leukämien, einschließlich akuter myeloischer Leukämie, chronischer myeloischer Leukämie, chronischer lymphatischer Leukämie, T-Zell-Lymphozyten-Leukämie und B-Zell-Lymphomen,
wobei die Verabreichung von 1) der Krebsimmuntherapie oder einem Impfstoff und 2) dem Wirkstoff, der in der Lage ist, die Reichhaltigkeit und Vielfalt des Darmmikrobioms des Subjekts zu erhöhen, zu einem oder mehreren von folgendem führt:
einer erhöhten Antitumorwirksamkeit der Krebsimmuntherapie oder des Impfstoffs,
einer stärkeren Immunantwort (z. B. erhöhte Anti-Tumor-T-Zellhäufigkeit unter PBMCs) und
verbesserter Hemmung des Tumorwachstums,
wobei das Mittel, das in der Lage ist, die Reichhaltigkeit und Vielfalt des Darmmikrobioms eines Subjekts zu erhöhen, ein ballaststoffhaltiges präbiotisches Mittel ist,
wobei das ballaststoffhaltige präbiotische Mittel eine gelbasierte InulinFormulierung ist, und
wobei die gelbasierte Inulinformulierung einen durchschnittlichen Polymerisationsgrad von 20 oder höher und von 47 oder weniger aufweist.

2. Mittel, das in der Lage ist, die Reichhaltigkeit und Vielfalt des Darmmikrobioms des menschlichen Subjekts zu erhöhen, zur Verwendung bei der Behandlung nach Anspruch 1, wobei die Verabreichung des Mittels, das in der Lage ist, die Reichhaltigkeit und Vielfalt des Darmmikrobioms des Subjekts zu erhöhen, vor, gleichzeitig mit und/oder nach der Verabreichung des Impfstoffs oder der Krebsimmuntherapie erfolgt.

3. Mittel, das in der Lage ist, die Reichhaltigkeit und Vielfalt des Darmmikrobioms des menschlichen Subjekts zu erhöhen, zur Verwendung bei der Behandlung nach Anspruch 1,
wobei die gelbasierte Inulinformulierung einen durchschnittlichen Polymerisationsgrad von etwa 28 aufweist.
wobei die gelbasierte Inulinformulierung eine oder mehrere präbiotische Verbindungen umfasst, die ausgewählt sind aus einem Fructo-Oligosaccharid, einem kurzkettigen Fructo-Oligosaccharid, einem Isomalt-Oligosaccharid, einem Transgalacto-Oligosaccharid, einem Pektin, einem Xylo-Oligosaccharid, einem Chitosan-Oligosaccharid, einem Beta-Glucan, einer modifizierten Stärke, einer resistenten Kartoffelstärke, Guarkernmehl, Bohnenkernmehl, Gelatine, Alginat, Glycerin, einer Polydextrose, einer D-Tagatose, einer Akazienfaser, Johannisbrot, Hafer und einer Zitrusfaser.

4. Mittel, das in der Lage ist, die Reichhaltigkeit und Vielfalt des Darmmikrobioms des menschlichen Subjekts zu erhöhen, zur Verwendung bei der Behandlung nach Anspruch 1, wobei die Krebsimmuntherapie einen oder mehrere Checkpoint-Inhibitoren (ICI) umfasst,
wobei der eine oder die mehreren ICI-Inhibitoren in der Lage sind, an einen oder mehrere der folgenden Rezeptoren zu binden, diese zu blockieren und/oder die Aktivität von einem oder mehreren von CTLA-4, PDL1, PDL2, PD1, BTLA, HVEM, TIM3, GAL9, LAG3, VISTA, KIR, 2B4, CD160 und CGEN-15049 zu hemmen,
wobei der eine oder die mehreren ICI-Inhibitoren ausgewählt sind aus Tremelimumab (CTLA-4-blockierender Antikörper), Anti-OX40, PD-L1-monoklonalem Antikörper (Anti-B7-H1; MEDI4736), MK-3475 (PD-1-Blocker), Nivolumab (Anti-PD1-Antikörper), CT-011 (Anti-PD1-Antikörper), monoklonalem Antikörper BY55, AMP224 (Anti-PDL1-Antikörper), BMS-936559 (Anti-PDL1-Antikörper), MPLDL3280A (Anti-PDL1-Antikörper), MSB0010718C (Anti-PDL1-Antikörper) und Yervoy/Ipilimumab (Anti-CTLA-4-Checkpoint-Inhibitor).

5. Mittel, das in der Lage ist, die Reichhaltigkeit und Vielfalt des Darmmikrobioms des menschlichen Subjekts zu erhöhen, zur Verwendung bei der Behandlung nach Anspruch 1, ferner umfassend die Verabreichung eines oder mehrerer chemotherapeutischer Mittel an das Subjekt, ausgewählt aus der Gruppe bestehend aus einem Alkylierungsmittel, einem Antimetaboliten, einem Anthrazyklin, einem Antitumor-Antibiotikum, einem monoklonalen Antikörper, einem Platinmittel, einem Pflanzenalkaloid, einem Topoisomerase-Inhibitor, einem Vinca-Alkaloid, einem Taxan und einem Epipodophyllotoxin.

6. Mittel, das in der Lage ist, die Reichhaltigkeit und Vielfalt des Darmmikrobioms eines menschlichen Subjekts zu erhöhen, zur Verwendung bei der Behandlung nach Anspruch 1, wobei die Verabreichung des Mittels, das in der Lage ist, die Reichhaltigkeit und Vielfalt des Darmmikrobioms des Subjekts zu erhöhen, zu einer erhöhten relativen Häufigkeit von Akkermansia, Lactobacillus, Ruminococcus, Roseburia und Butyricicoccus innerhalb des Darmmikrobioms des Subjekts führt.

## Revendications

1. Agent capable d'augmenter la richesse et la diversité du microbiome intestinal d'un sujet humain pour une utilisation dans le traitement d'un cancer, le traitement comprenant l'administration à un sujet humain souffrant d'un cancer
1) d'une immunothérapie contre le cancer ou d'un vaccin, et
2) de l'agent capable d'augmenter la richesse et la diversité du microbiome intestinal du sujet humain,
dans lequel l'agent capable d'augmenter la richesse et la diversité du microbiome intestinal du sujet est administré par voie orale,
dans lequel le vaccin est un vaccin pour traiter ou prévenir le cancer,
dans lequel le cancer est un ou plusieurs cancers parmi le cancer du sein, de l'ovaire, de la prostate, du poumon, du rein, de l'estomac, du côlon, du testicule, de la tête et du cou, du pancréas, du cerveau, le mélanome et d'autres tumeurs d'organes tissulaires et tumeurs hématologiques, telles que les lymphomes et les leucémies, y compris la leucémie myéloïde aiguë, la leucémie myéloïde chronique, la leucémie lymphoïde chronique, la leucémie lymphoïde à cellules T et les lymphomes à cellules B,
dans lequel l'administration 1) de l'immunothérapie contre le cancer ou d'un vaccin, et 2) de l'agent capable d'augmenter la richesse et la diversité du microbiome intestinal du sujet, entraîne une ou plusieurs parmi :
une efficacité antitumorale accrue de l'immunothérapie contre le cancer ou du vaccin,
une réponse immunitaire plus forte (par exemple, augmentation de la fréquence de cellule T antitumorale parmi les PBMC) et une inhibition accrue de la croissance tumorale,
dans lequel l'agent capable d'augmenter la richesse et la diversité du microbiome intestinal d'un sujet est un agent prébiotique contenant des fibres,
dans lequel l'agent prébiotique contenant des fibres est une formulation d'inuline à base de gel, et
dans lequel la formulation d'inuline à base de gel a un degré moyen de polymérisation supérieur ou égal à 20 et inférieur ou égal à 47.

2. Agent capable d'augmenter la richesse et la diversité du microbiome intestinal d'un sujet humain pour une utilisation dans le traitement selon la revendication 1, dans lequel l'administration de l'agent capable d'augmenter la richesse et la diversité du microbiome intestinal du sujet a lieu avant, en même temps que et/ou après l'administration du vaccin ou de l'immunothérapie contre le cancer.

3. Agent capable d'augmenter la richesse et la diversité du microbiome intestinal d'un sujet humain pour une utilisation dans le traitement selon la revendication 1,
dans lequel la formulation d'inuline à base de gel a un degré moyen de polymérisation d'environ 28,
dans lequel la formulation d'inuline à base de gel comprend un ou plusieurs composés prébiotiques choisis parmi un fructo-oligosaccharide, un fructo-oligosaccharide à chaîne courte, un isomalto-oligosaccharide, un transgalacto-oligosaccharide, une pectine, un xylo-oligosaccharide, un chitosane-oligosaccharide, un bêta-glucane, de la gomme arabique, un amidon modifié, un amidon de pomme de terre résistant, de la gomme de guar, de la gomme de haricot, de la gélatine, de l'alginate, du glycérol, un polydextrose, un D-tagatose, une fibre d'acacia, de la caroube, de l'avoine et une fibre d'agrume.

4. Agent capable d'augmenter la richesse et la diversité du microbiome intestinal d'un sujet humain pour une utilisation dans le traitement selon la revendication 1, dans lequel l'immunothérapie contre le cancer comprend un ou plusieurs inhibiteurs de point de contrôle immunitaire (ICI),
dans lequel le ou les plusieurs inhibiteurs ICI sont capables de se lier à, de bloquer et/ou d'inhiber l'activité d'un ou de plusieurs parmi CTLA-4, PDL1, PDL2, PD1, BTLA, HVEM, TIM3, GAL9, LAG3, VISTA, KIR, 2B4, CD160 et CGEN-15049,
dans lequel le ou les plusieurs inhibiteurs ICI sont choisis parmi le trémélimumab (anticorps de blocage de CTLA-4), anti-OX40, l'anticorps monoclonal PD-L1 (anti-B7-H1 ; MEDI4736), MK-3475 (bloqueur de PD-1), le nivolumab (anticorps anti-PD1), CT-011 (anticorps anti-PD1), l'anticorps monoclonal BY55, AMP224 (anticorps anti-PDL1), BMS-936559 (anticorps anti-PDL1), MPLDL3280A (anticorps anti-PDL1), MSB0010718C (anticorps anti-PDL1) et Yervoy/ipilimumab (inhibiteur de point de contrôle anti-CTLA-4).

5. Agent capable d'augmenter la richesse et la diversité du microbiome intestinal d'un sujet humain pour une utilisation dans le traitement selon la revendication 1, comprenant en outre l'administration au sujet d'un ou de plusieurs agents chimiothérapeutiques choisis dans le groupe constitué par un agent alkylant, un antimétabolite, une anthracycline, un antibiotique antitumoral, un anticorps monoclonal, un agent de platine, un alcaloïde végétal, un inhibiteur de la topoisomérase, un alcaloïde de la pervenche, un taxane et une épipodophyllotoxine.

6. Agent capable d'augmenter la richesse et la diversité du microbiome intestinal d'un sujet humain pour une utilisation dans le traitement selon la revendication 1, dans lequel l'administration de l'agent capable d'augmenter la richesse et la diversité du microbiome intestinal du sujet entraîne une augmentation de l'abondance relative d'Akkermansia, de Lactobacillus, de Ruminococcus, de Roseburia et de Butyricicoccus dans le microbiome intestinal du sujet.
